(19) Europäisches Patentamt
European Patent Office
Office européen des brevets

(11) EP 1 061 081 B1

(12) **FASCICULE DE BREVET EUROPEEN**

(45) Date de publication et mention de la délivrance du brevet:
**17.04.2002 Bulletin 2002/16**

(21) Numéro de dépôt: **00401685.3**

(22) Date de dépôt: **15.06.2000**

(51) Int Cl.[7]: **C07D 491/052**, A61K 31/436, A61P 35/00 // (C07D491/052, 311:00, 221:00)

(54) **Nouveaux dérivés de carboxylate de 7-oxo-2,3,7,14-tétrahydro-1H-benzo(b)pyrano(3,2-h)acridine, leur procédé de préparation et les compositions pharmaceutiques qui les contiennent**

7-Oxo-2,3,7,14-tetrahydro-1H-benzo[b]pyrano[3,2-h]acridincarboxylatderivate, Verfahren zu ihrer Herstellung und diese enthaltende pharmazeutische Zubereitungen

7-Oxo-2,3,7,14-tetrahydro-1H-benzo[b]pyrano[3,2-h]acridine carboxylate derivatives, process for their preparation and pharmaceutical compositions containing them

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **16.06.1999 FR 9907611**

(43) Date de publication de la demande:
**20.12.2000 Bulletin 2000/51**

(73) Titulaire: **LES LABORATOIRES SERVIER**
**92200 Neuilly sur Seine (FR)**

(72) Inventeurs:
- **Koch, Michel**
  **78170 La Celle Saint Cloud (FR)**
- **Tillequin, Francois**
  **75014 Paris (FR)**
- **Michel, Sylvie**
  **75002 Paris (FR)**
- **Atassi, Ghanem**
  **92210 Saint Cloud (FR)**
- **Pierre, Alain**
  **78580 Les Alluets le Roi (FR)**
- **Renard, Pierre**
  **78150 Le Chesnay (FR)**
- **Pfeiffer, Bruno**
  **95320 Saint Leu la Foret (FR)**

(56) Documents cités:
**WO-A-99/32491**

- **A ELOMRI ET AL: "Synthesis and cytotoxic and antitumor activity of esters in the 1,2-dihdroxy-1,2-dihydroacronycine series" JOURNAL OF MEDICINAL CHEMISTRY,US,AMERICAN CHEMICAL SOCIETY. WASHINGTON, vol. 24, no. 39, 1 janvier 1996 (1996-01-01), pages 4762-4764, XP002076704 ISSN: 0022-2623**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 1 061 081 B1

## Description

La présente invention concerne de nouveaux dérivés de carboxylate de 7-oxo-2,3,7,14-tétrahydro-1*H*-benzo [*b*]pyrano[3,2-*h*]acridine, leur procédé de préparation et les compositions pharmaceutiques qui les contiennent.

Les composés de l'invention constituent des dérivés de l'acronycine qui est un alcaloïde présentant des propriétés antitumorales mises en évidence dans des modèles expérimentaux (*J. Pharm.. Sci.,* 1966, 55 (8), 758-768). Cependant, malgré un large spectre d'activité, l'acronycine a une faible solubilité limitant sa biodisponibilité ainsi que son utilisation dans des compositions pharmaceutiques administrables par voie d'injection.

Diverses modifications ont été réalisées sur cette molécule, comme celles décrites dans *J. Med. Chem.,* 1996, 39, 4762-4766, et qui ont permis de résoudre quelques difficultés liées au problème de solubilité de ces produits. Néanmoins, les besoins de la thérapeutique anticancéreuse exigent le développement constant de nouveaux agents antitumoraux, dans le but d'obtenir des médicaments à la fois plus actifs et mieux tolérés. Plus particulièrement, les tumeurs solides posent un problème majeur à la chimiothérapie anticancéreuse, de par leur résistance intrinsèque et/ou acquise, aux produits existants.

Les composés de l'invention, outre le fait qu'ils soient nouveaux, présentent une activité in-vivo et in-vitro surprenante et supérieure à celle observée jusqu'ici. De plus, lesdits composés possèdent des propriétés de solubilisation intéressantes et adaptées à une administration des produits sous forme liquide. Ainsi, les composés découverts par la Demanderesse possèdent des propriétés antitumorales qui les rendent particulièrement utiles pour le traitement des cancers et notamment des tumeurs solides.

Plus particulièrement, la présente invention concerne les composés de formule (I) :

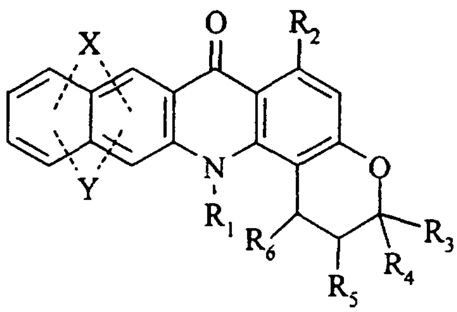

**(I)**

dans laquelle :

X, Y, identiques ou différents, indépendamment l'un de l'autre, représentent un groupement choisi parmi atome d'hydrogène, d'halogène, groupement hydroxy, mercapto, cyano, nitro, alkyle ($C_1$-$C_6$) linéaire ou ramifié, alkoxy ($C_1$-$C_6$) linéaire ou ramifié, trihalogénoalkyle ($C_1$-$C_6$) linéaire ou ramifié, amino éventuellement substitué par un ou deux groupements, identiques ou différents, alkyle ($C_1$-$C_6$) linéaires ou ramifiés eux-mêmes éventuellement substitués par un groupement alkoxy ($C_1$-$C_6$) linéaire ou ramifié ou par un groupement de formule $-NR_7R_8$ dans laquelle $R_7$ et $R_8$, identiques ou différents, indépendamment l'un de l'autre, représentent un atome d'hydrogène, un groupement alkyle ($C_1$-$C_6$) linéaire ou ramifié, aryle ou arylalkyle ($C_1$-$C_6$) linéaire ou ramifié, ou forment ensemble un groupement méthylènedioxy ou un groupement éthylènedioxy, étant entendu que les substituants X et Y peuvent être présents sur l'un ou l'autre des deux cycles benzéniques adjacents,

$R_1$ représente un atome d'hydrogène ou un groupement alkyle ($C_1$-$C_6$) linéaire ou ramifié,

$R_2$ représente :

- -un atome d'hydrogène,
- -un groupement hydroxy,
- -un groupement alkyle ($C_1$-$C_6$) linéaire ou ramifié,
- -un groupement alkoxy ($C_1$-$C_6$) linéaire ou ramifié, éventuellement substitué par un groupement choisi parmi :

  * groupement de formule $NR_9R_{10}$ dans laquelle $R_9$ et $R_{10}$, identiques ou différents, indépendamment l'un de l'autre, représentent un atome d'hydrogène, un groupement alkyle ($C_1$-$C_6$) linéaire

ou ramifié, ou un groupement hydroxyalkyle ($C_1$-$C_6$) linéaire ou ramifié,

* et hétérocycle saturé ou insaturé, monocyclique ou bicyclique, de 5 à 7 chaînons comportant un ou deux hétéroatomes choisis parmi oxygène, azote et soufre,

-un groupement alkylcarbonyloxy ($C_1$-$C_6$) linéaire ou ramifié,
-ou un groupement amino éventuellement substitué :

* par un ou deux groupements, identiques ou différents, alkyle ($C_1$-$C_6$) linéaire ou ramifié, aryle, ou arylalkyle ($C_1$-$C_6$) linéaire ou ramifié,
* par un groupement alkylcarbonyle ($C_1$-$C_6$) linéaire ou ramifié, éventuellement substitué par un groupement $-NR_7R_8$ avec $R_7$ et $R_8$ tels que définis précédemment,
* par un groupement de formule $-R_{11}-NR_9R_{10}$, dans laquelle $R_{11}$ représente un groupement alkylène ($C_1$-$C_6$) linéaire ou ramifié et $R_9$, $R_{10}$, identiques ou différents, indépendamment l'un de l'autre représentent un atome d'hydrogène, un groupement alkyle ($C_1$-$C_6$) linéaire ou ramifié, ou un groupement hydroxyalkyle ($C_1$-$C_6$) linéaire ou ramifié,
* par un groupement alkylène ($C_1$-$C_6$) linéaire ou ramifié, substitué par un hétérocycle saturé ou insaturé, monocyclique ou bicyclique, de 5 à 7 chaînons comportant un ou deux hétéroatomes choisis parmi oxygène, azote et soufre,
* ou par un groupement de formule $-R_{11}-CO-R_{12}$ dans laquelle $R_{11}$ est tel que défini précédemment, et $R_{12}$ représente un groupement hydroxy ou alkoxy ($C_1$-$C_6$) linéaire ou ramifié,

$R_3$, $R_4$, identiques ou différents, indépendamment l'un de l'autre, représentent un atome d'hydrogène ou un groupement alkyle ($C_1$-$C_6$) linéaire ou ramifié,

$R_5$ et/ou $R_6$ représente(nt) un groupement de formule -O-CO-U-V dans laquelle :

-U représente une chaîne alkylène ($C_1$-$C_8$) linéaire ou ramifié, éventuellement substituée par un ou plusieurs groupements, identiques ou différents, choisis parmi aryle, hydroxy et alkoxy ($C_1$-$C_6$) linéaire ou ramifié,
-V représente un groupement choisi parmi :

* carboxy,
* $-CO_2R_{13}$ dans lequel $R_{13}$ représente un groupement alkyle ($C_1$-$C_6$) linéaire ou ramifié (éventuellement substitué par un ou plusieurs groupements hydroxy, aryle ou arylalkyle ($C_1$-$C_6$) linéaire ou ramifié,
* hydroxy,
* alkoxy ($C_1$-$C_6$) linéaire ou ramifié,
* $-NR_7R_8$ dans lequel $R_7$ et $R_8$, identiques ou différents, sont tels que définis précédemment,
* $-NR_7-CO_2R_{13}$ dans lequel $R_7$ et $R_{13}$ sont tels que définis précédemment,
* $-NR_7-COR_{13}$ dans lequel $R_7$ et $R_{13}$ sont tels que définis précédemment,
* $-COR_{13}$ dans lequel $R_{13}$ est tel que défini précédemment,
* et $-CO-NR_7R_8$ dans lequel $R_7$ et $R_8$, identiques ou différents, sont tels que définis précédemment,

et dans le cas où un seul des deux groupements $R_5$ et $R_6$ représente un groupement de formule -O-CO-U-V, alors l'autre desdits groupements $R_5$ ou $R_6$ représente un groupement
Z choisi parmi :

- hydroxy,
- alkoxy ($C_1$-$C_6$) linéaire ou ramifié,
- alkylcarbonyloxy ($C_1$-$C_6$) linéaire ou ramifié,
- arylcarbonyloxy,
- arylalkylcarbonyloxy ($C_1$-$C_6$) linéaire ou ramifié,
- et amino éventuellement substitué par un ou deux groupements alkyle ($C_1$-$C_6$) linéaires ou ramifiés, identiques ou différents,

leurs isomères optiques, N-oxydes, ainsi que leurs sels d'addition à un acide ou à une base pharmaceutiquement acceptable.

Etant entendu que par "aryle", on comprend un groupement phényle ou naphtyle, comportant éventuellement une ou plusieurs substitutions, identiques ou différentes, choisies parmi hydroxy, halogéno, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, la lysine, etc...

Parmi les bases pharmaceutiquement acceptables, on peut citer à titre non limitatif l'hydroxyde de sodium, l'hydroxyde de potassium, la triéthylamine, la tertbutylamine, etc...

Les substituants $R_3$ et $R_4$ préférés selon l'invention sont les groupements alkyle ($C_1$-$C_6$) linéaires ou ramifiés, avec $R_3$ et $R_4$ identiques ou différents. D'une façon préférentielle, $R_3$ et $R_4$ sont identiques et représentent chacun un groupement méthyle.

Les substituants $R_2$ préférés selon l'invention sont les groupements alkoxy ($C_1$-$C_6$) linéaires ou ramifiés, ou les groupements amino éventuellement substitués par un ou deux substituants tels que définis dans la formule (I).

Selon une variante avantageuse, les composés préférés de l'invention sont ceux pour lesquels $R_5$ représente un groupement de formule -O-CO-U-V dans laquelle U et V sont tels que définis dans la formule (I), et $R_6$ représente un groupement Z tel que défini dans la formule (I).

Selon une autre variante avantageuse, les composés préférés de l'invention sont ceux pour lesquels $R_6$ et $R_5$ représentent chacun un groupement de formule -O-CO-U-V identique dans laquelle U et V sont tels que définis dans la formule (I).

Le substituant $R_5$ préféré selon l'invention est le groupement de formule -O-CO-U-V dans laquelle U représente une chaîne alkylène ($C_1$-$C_4$) linéaire et V représente un groupement choisi parmi carboxy, $-NR_7R_8$, $-NR_7-CO_2R_{13}$ et $-NR_7-COR_{13}$ dans lesquels $R_7$, $R_8$ et $R_{13}$, identiques ou différents, représente un atome d'hydrogène ou un groupement alkyl ($C_1$-$C_6$) linéaire ou ramifié.

Le substituant $R_6$ préféré selon l'invention est le groupement alkylcarbonyloxy ($C_1$-$C_6$) linéaire ou ramifié.

Les composés préférés de l'invention sont le :

- acide (±)-*cis*-4-{[1-(acétyloxy)-6-méthoxy-3,3,14-triméthyl-7-oxo-2,3,7,14-tétrahydro-1*H*-benzo[b]pyrano[3,2-h]acridin-2-yl]oxy}-4-oxobutanoïque,
- acide (±)-*cis*-4-({1-[(3-carboxypropanoyl)oxy]-6-méthoxy-3,3,14-triméthyl-7-oxo-2,3,7,14-tétrahydro-1*H*-benzo[*b*]pyrano[3,2-*h*]acridin-2-yl}oxy)-4-oxobutanoïque,
- acide (±)-*cis*-5-{[1-(acétyloxy)-6-méthoxy-3,3,14-triméthyl-7-oxo-2,3,7,14-tétrahydro-1*H*-benzo[b]pyrano[3,2-h]acridin-2-yl]oxy}-5-oxopentanoïque,
- *cis*-[1-(acétyloxy)-6-méthoxy-3,3,14-triméthyl-7-oxo-2,3,7,14-tétrahydro-1*H*-benzo[*b*] pyrano[3,2-*h*]acridin-2-yl] (diméthylamino)acétate,
- et *cis*-[1-(acétyloxy)-6-méthoxy-3,3,14-triméthyl-7-oxo-2,3,7,14-tétrahydro-1*H*-benzo[*b*] pyrano[3,2-*h*]acridin-2-yl]-4-(diméthylamino)butanoate.

Les isomères optiques, N-oxydes, ainsi que les sels d'addition à un acide ou à une base, pharmaceutiquement acceptable, des composés préférés font partie intégrante de l'invention.

La présente invention s'étend également au procédé de préparation des composés de formule (I), caractérisé en ce que l'on fait réagir :

- *soit un dérivé de l'acide 3-amino-2-naphtalène carboxylique (II) :*

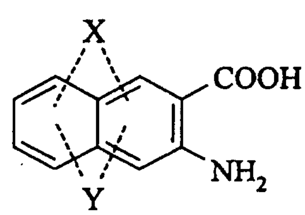

**(II)**

dans laquelle X et Y sont tels que définis dans la formule (I), avec un dérivé du phloroglucinol de formule (III) :

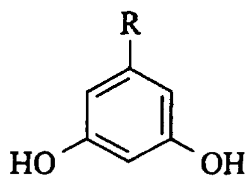

(III)

dans laquelle R représente un atome d'hydrogène, un groupement hydroxy ou un groupement alkyle ($C_1$-$C_6$) linéaire ou ramifié,
pour conduire aux composés de formule (IV) :

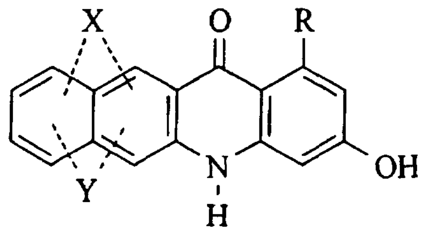

(IV)

dans laquelle X, Y et R sont tels que définis précédemment,
qui sont ensuite traités en condition basique dans un solvant aprotique tel que la diméthylformamide, par un alcyne de formule (V) :

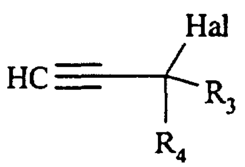

(V)

dans laquelle Hal représente un atome d'halogène et $R_3$ et $R_4$ sont tels que définis dans la formule (I),
pour conduire aux composés de formule (VI) :

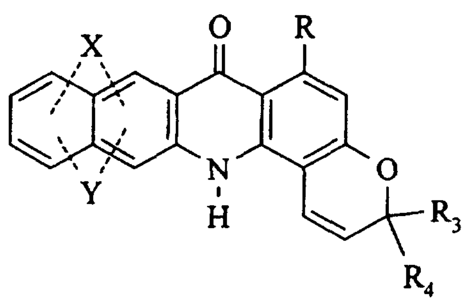

(VI)

dans laquelle X, Y, R, $R_3$ et $R_4$ sont tels que définis précédemment,

- *soit un dérivé de l'acide 3-halogéno-2-naphtalène carboxylique de formule (VII) :*

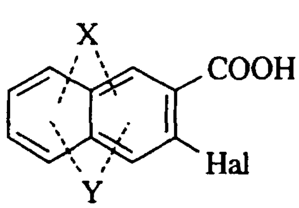

(VII)

dans laquelle X et Y sont tels que définis dans la formule (I) et Hal représente un atome d'halogène, tel que le chlore ou le brome,
avec un dérivé amino-chromène de formule (VIII) :

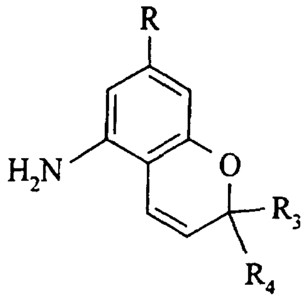

(VIII)

dans laquelle $R_3$ et $R_4$ sont tels que définis dans la formule (I) et R a la même signification que précédemment, pour conduire également aux composés de formule (VI) :

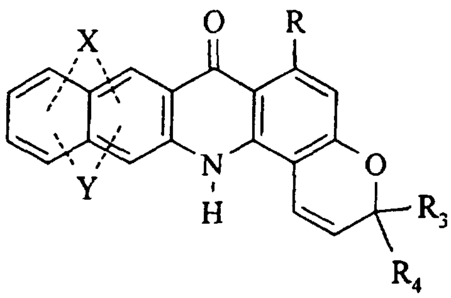

(VI)

dans laquelle X, Y, R, $R_3$ et $R_4$ sont tels que définis précédemment,
composés de formule (VI) dont l'atome d'azote est éventuellement substitué, par action d'un halogénure d'alkyle ou d'un sulfate de dialkyle en présence d'un agent de déprotonation, tel que l'hydrure de sodium, en solvant polaire aprotique, permettant d'obtenir les composés de formule (IX) :

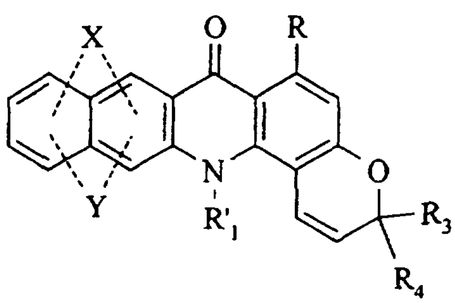

(IX)

dans laquelle X, Y, R, $R_3$ et $R_4$ sont tels que définis précédemment, et $R'_1$ représente un groupement alkyle ($C_1$-$C_6$) linéaire ou ramifié,
composés de formule (IX) qui sont soumis à l'action d'un agent alkylant tel qu'un sulfate de dialkyle, ou d'un agent acylant, pour conduire aux composés de formule (X) :

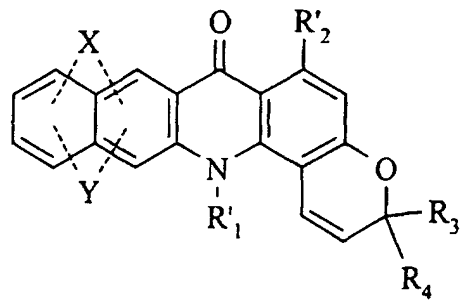

**(X)**

dans laquelle X, Y, R'$_1$, $R_3$ et $R_4$ sont tels que définis précédemment et R'$_2$ représente un groupement alkoxy (éventuellement substitué par un groupement de formule $NR_9R_{10}$ dans laquelle $R_9$ et $R_{10}$ sont tels que définis dans la formule (I)), ou alkylcarbonyloxy ($C_1$-$C_6$) linéaire ou ramifié,
composé de formule (X) qui est traité, dans le cas où R'$_2$ représente un groupement alkoxy par exemple, par un composé de formule (XI) :

HNRaRb **(XI)**

dans laquelle Ra représente un atome d'hydrogène, un groupement alkyle ($C_1$-$C_6$) linéaire ou ramifié, aryle, ou arylalkyle ($C_1$-$C_6$) linéaire ou ramifié, et Rb représente un atome d'hydrogène, un groupement alkyle ($C_1$-$C_6$) linéaire ou ramifié, aryle, arylalkyle ($C_1$-$C_6$) linéaire ou ramifié, un groupement de formule $-R_{11}-NR_9R_{10}$ dans laquelle $R_{11}$, $R_{10}$ et $R_9$ sont tels que définis dans la formule (I), un groupement alkylcarbonyle ($C_1$-$C_6$) linéaire ou ramifié la partie alkyle étant éventuellement substituée par un groupement $NR_7R_8$ tel que défini dans la formule (I), un groupement hétérocycloalkylène (les termes alkylène et hétérocycle ayant la même signification que dans la formule (I)) ou un groupement de formule $-R_{11}-CO-R_{12}$ dans laquelle $R_{11}$ et $R_{12}$ ont la même définition que dans la formule (I),
pour conduire aux composés de formule (XII) :

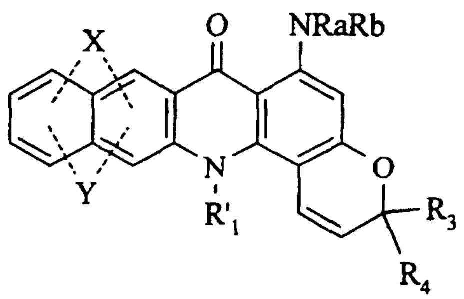

**(XII)**

dans laquelle X, Y, R'$_1$, $R_3$, $R_4$, Ra et Rb sont tels que définis précédemment,
l'ensemble des composés de formule (VI), (IX), (X) et (XII) formant les composés de formule (XIII) :

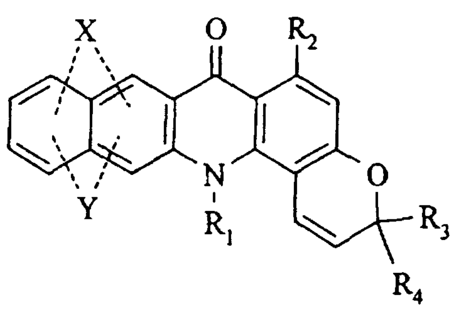

**(XIII)**

dans laquelle X, Y, $R_1$, $R_2$, $R_3$ et $R_4$ ont la même signification que dans la définition générale de la formule (I),
composés de formule (XIII) qui sont soumis,

a) ➪ soit à l'action du tétroxyde d'osmium en milieu polaire et en présence de 4-méthylmorpholine-N-oxyde, pour conduire aux composés de formules (XIV/a) et (XIV/b) :

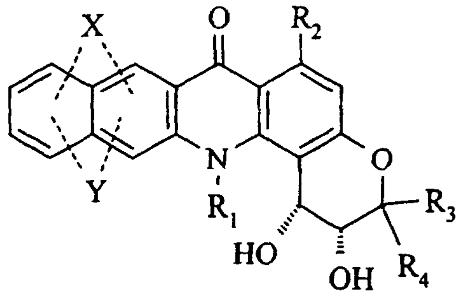

(XIV/a)

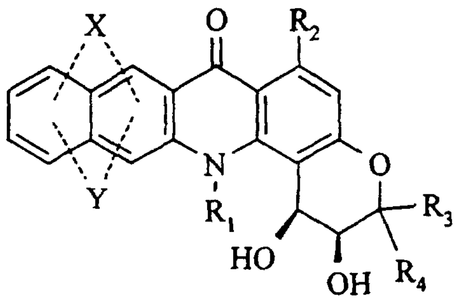

(XIV/b)

dans lesquelles X, Y, $R_1$, $R_2$, $R_3$ et $R_4$ sont tels que définis précédemment,
les composés de formule (XIV/a) et (XIV/b) pouvant aussi être obtenus séparément par synthèse chirale et notamment par *cis* dihydroxylation asymétrique à partir du composé (XIII) en utilisant des ligands chiraux du type pyridine ou phtalazine bisubstitués par des alcaloïdes de *Cinchona* comme la dihydroquinine et son diastéréoisomère dextrogyre la dihydroquinidine,
b) ➪ soit à l'action du permanganate de potassium en milieu polaire, pour conduire aux composés de formule (XV) :

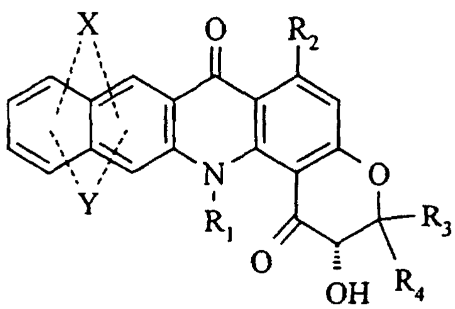

(XV)

dans laquelle X, Y, $R_1$, $R_2$, $R_3$ et $R_4$ sont tels que définis précédemment,
composés de formule (XV) qui sont soumis à des conditions réductrices en présence de $NaBH_4$ par exemple, pour conduire aux composés de formule (XIV/c) :

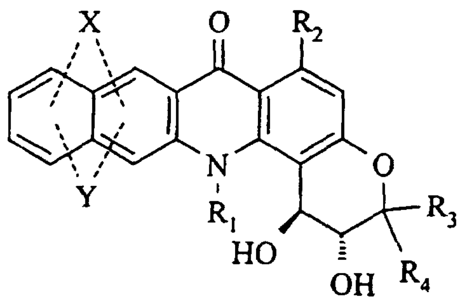

**(XIV/c)**

dans laquelle X, Y, $R_1$, $R_2$, $R_3$ et $R_4$ sont tels que définis précédemment,
l'ensemble des composés de formules (XIV/a), (XIV/b) et (XIV/c) formant les composés de formule (XIV) :

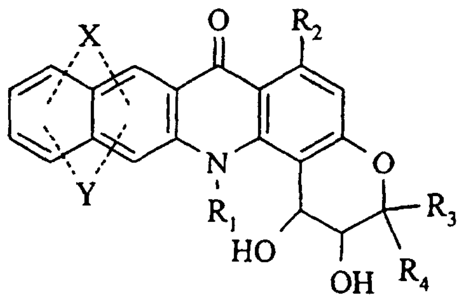

**(XIV)**

dans laquelle X, Y, $R_1$, $R_2$, $R_3$ et $R_4$ sont tels que définis précédemment,
composés de formule (XIV) qui sont soumis :

- soit à l'action d'un alcool de formule $R_{20}$-OH dans laquelle $R_{20}$ représente un groupement alkyle ($C_1$-$C_6$) linéaire ou ramifié,

pour conduire aux composés de formule (XVI/a) :

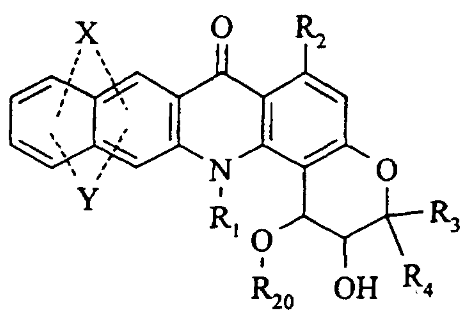

**(XVI/a)**

dans laquelle X, Y, $R_1$, $R_2$, $R_3$, $R_4$ et $R_{20}$ sont tels que définis précédemment,
composés de formule (XVI/a) dont la fonction alcool est estérifiée en présence d'un composé de formule

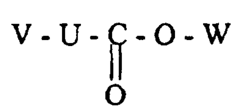

ou $(V\text{-}U\text{-}CO)_2O$ dans lesquelles U et V sont tels que définis dans la formule (I) et W représente un groupement partant,
pour conduire aux composés de formule (I/a), cas particulier des composés de formule (I) :

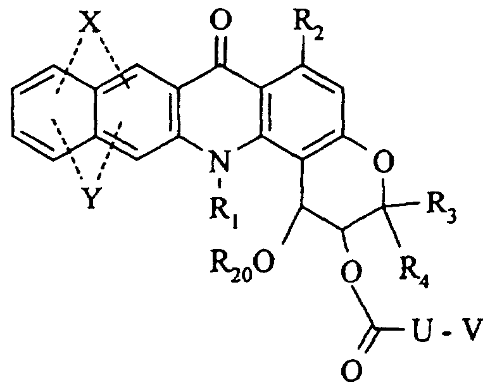

**(I/a)**

dans laquelle X, Y, $R_1$, $R_2$, $R_3$, $R_4$, $R_{20}$, U et V sont tels que définis précédemment,

✧ soit à l'action d'un iodure d'alkyle de formule $R'_{20}$-I dans laquelle $R'_{20}$ représente un groupement alkyle ($C_1$-$C_6$) linéaire ou ramifié, en présence de sel d'Argent, pour conduire aux composés de formule (XVI/b) :

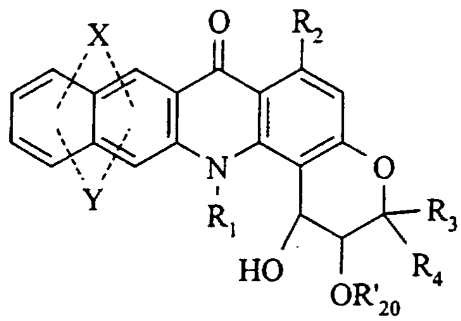

**(XVI/b)**

cas particulier des composés de formule (I), dans laquelle X, Y, $R_1$, $R_2$, $R_3$, $R_4$ et $R'_{20}$ sont tels que définis précédemment,
composés de formule (XVI/b) dont la fonction alcool est estérifiée en présence d'un composé de formule

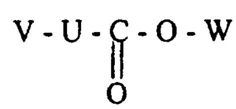

ou $(V\text{-}U\text{-}CO)_2O$ tels que définis précédemment, pour conduire aux composés de formule (I/b) :

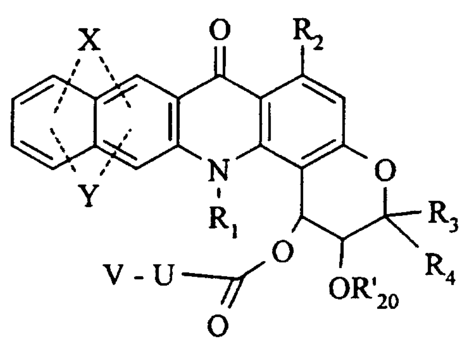

**(I/b)**

cas particulier des composés de formule (I), dans laquelle X, Y, $R_1$, $R_2$, $R_3$, $R_4$, $R'_{20}$, U et V sont tels que définis précédemment,

- soit à l'action directe d'un composé de formule

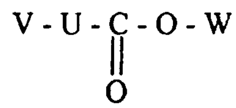

ou $(V\text{-}U\text{-}CO)_2O$, tels que définis précédemment, en présence d'une base, telle que la triéthylamine ou la 4-diméthylaminopyridine, afin d'obtenir les composés de formule (I/c), cas particulier des composés de formule (I) :

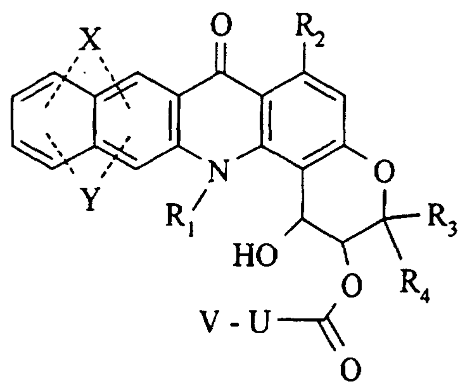

(I/c)

dans laquelle X, Y, $R_1$, $R_2$, $R_3$, $R_4$, U et V sont tels que définis précédemment, composé de formule (I/c), qui peut être soumis à nouveau, dans les mêmes conditions opératoires, à l'action d'un anhydride de formule $(R_{30}CO)_2O$ dans laquelle $R_{30}$ représente un groupement alkyle ($C_1$-$C_6$) linéaire ou ramifié, aryle ou arylalkyle ($C_1$-$C_6$) linéaire ou ramifié, pour conduire aux composés de formule (I/d) :

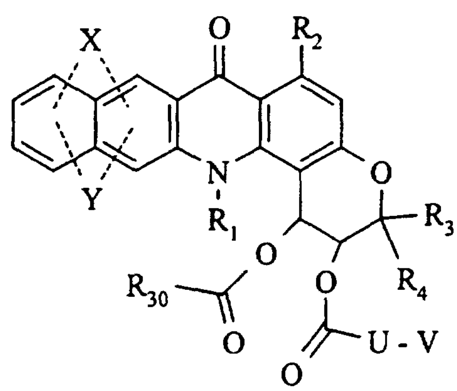

(I/d)

dans laquelle X, Y, $R_1$, $R_2$, $R_3$, $R_4$, $R_{30}$, U et V sont tels que définis précédemment,

ou composé de formule (I/c) qui peut être traité de nouveau avec un composé de formule

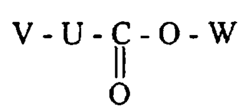

ou $(V\text{-}U\text{-}CO)_2O$ tels que définis précédemment, pour conduire aux composés de formule (I/e), cas particulier des composés de formule (I) :

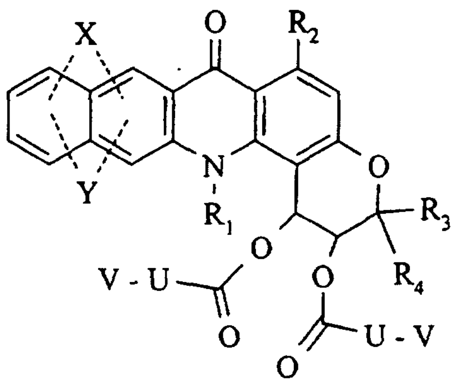

(I/e)

dans laquelle X, Y, $R_1$, $R_2$, $R_3$, $R_4$, U et V ont la même signification que dans la formule (I), étant entendu que les deux groupements U et les deux groupements V peuvent être chacun identique ou différent,

c) ⇨ soit à l'action d'un peracide, comme l'acide m-chloroperbenzoïque, pour conduire au composé de formule (XVII) :

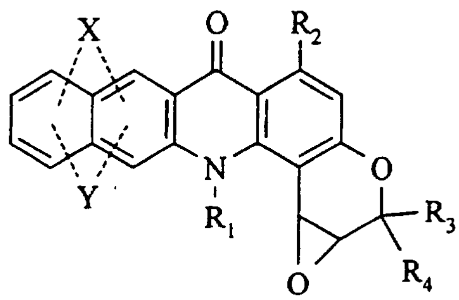

(XVII)

dans laquelle X, Y, $R_1$, $R_2$, $R_3$ et $R_4$, sont tels que définis précédemment,

composé de formule (XVII) qui est traité éventuellement par de l'ammoniaque ou une amine primaire ou secondaire pour conduire aux composés de formule (XVIII/a) et/ou (XVIII/b) suivant la nature des réactifs :

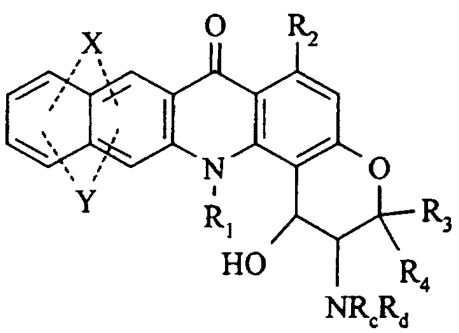

(XVIII/a)

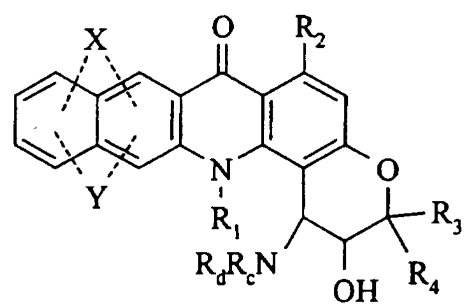

(XVIII/b)

dans lesquelles X, Y, $R_1$, $R_2$, $R_3$ et $R_4$, sont tels que définis précédemment, et $R_c$ et $R_d$ représentent un atome d'hydrogène ou un groupement alkyle ($C_1$-$C_6$) linéaire ou ramifié,

composés de formules (XVIII/a) et (XVIII/b) qui sont mis en présence d'un composé de formule

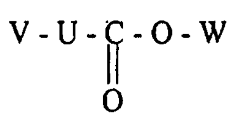

tel que défini précédemment, pour conduire respectivement aux composés de formules (I/f) et (I/g), cas particuliers des composés de formule (I) :

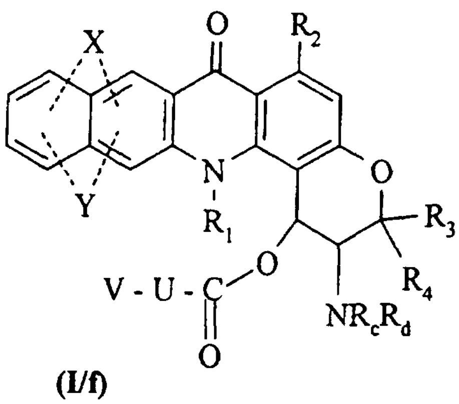

(I/f)

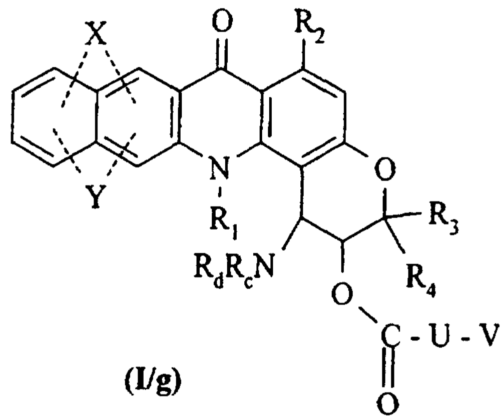

(I/g)

dans lesquelles X, Y, $R_1$, $R_2$, $R_3$, $R_4$, $R_c$, $R_d$, U et V sont tels que définis précédemment,
les composés (I/a) à (I/g) formant l'ensemble des composés de l'invention qui sont purifiés, le cas échéant, selon une technique classique de purification, qui peuvent, si on le désire, être séparés en leurs différents isomères optiques selon une technique classique de séparation et qui sont transformés, si on le souhaite, en leurs N-oxydes et, le cas échéant, en leurs sels d'addition à un acide ou à une base pharmaceutiquement acceptable.

Les composés de formule (II), (III), (V), (VII), (VIII) et (XI) sont soit des composés commerciaux, soit obtenus selon les méthodes classiques de la synthèse organique. Les composés de formule (VIII) sont notamment obtenus selon les conditions décrites dans *Chem. Ber.* 1978, 191, 439. La réaction de condensation entre les composés de formule (VII) et les composés de formule (VIII) est notamment décrite dans la revue *Heterocycles*, 1992, 34(4), 799-806.
Les composés de formule (I) présentent des propriétés antitumorales particulièrement intéressantes. Ils ont un excellente cytotoxicité in vitro sur des lignées cellulaires, issues de tumeurs murines et humaines, due à un blocage spécifique du cycle cellulaire, et sont actifs in vivo, chez la souris, sur des tumeurs transplantables murines et humaines. Les propriétés caractéristiques de ces composés permettent leur utilisation en thérapeutique en tant qu'agents antitumoraux.
La présente invention a également pour objet les compositions pharmaceutiques contenant comme principe actif au moins un composé de formule (I), ses isomères optiques, N-oxydes ou un de ses sels d'addition à une base ou un acide pharmaceutiquement acceptable, seul ou en combinaison avec un ou plusieurs excipients ou véhicules inertes, non toxiques, pharmaceutiquement acceptables.
Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...
La posologie utile varie selon l'âge et le poids du patient, la voie d'administration, la nature et la sévérité de l'affection et la prise de traitements éventuels associés et s'échelonne de 0,5 mg à 500 mg en une ou plusieurs prises par jour.
Les exemples suivants illustrent l'invention mais ne la limitent en aucune façon.
Les produits de départ utilisés sont des produits connus ou préparés selon des modes opératoires connus.
Les structures des composés décrits dans les exemples et les préparations ont été déterminées selon les techniques spectrophotométriques usuelles (infrarouge, résonance magnétique nucléaire,..).

**PREPARATION A : (±)*cis*-1,2-Dihydroxy-6-méthoxy-3,3,14-triméthyl-2,3,7,14-tétrahydro-1*H*-benzo[*b*]pyrano[3,2-*h*]acridin-7-one**

***Stade A* : *1,3-Dihydroxy-5,12-dihydro-benzo[b]acridin-12-one***

A une solution de 5 g d'acide 3-amino-2-naphtalène carboxylique dans 50 ml d'heptane-1-ol sont additionnés

3,5 g de 1,3,5-trihydroxybenzène et 62,5 mg d'acide paratoluène sulfonique. Le mélange est maintenu sous agitation pendant 48 heures à reflux avec un Dean Stark, puis le mélange réactionnel est concentré sous vide. Le résidu est chromatographié sur gel de silice (éluant : cyclohexane/acétone : 90/10). Le produit isolé est cristallisé dans un mélange cyclohexane/acétone et permet d'obtenir 5,2 g du produit attendu.

***Stade B** : 6-Hydroxy-3,3-diméthyl-7,14-dihydro-3H-benzo[b]pyrano[3,2-h]acridin-7-one*

A une solution de 2 g du produit du stade A dans 50 ml de diméthylformamide anhydre, sous atmosphère inerte, sont additionnés 2 g de carbonate de potassium anhydre. Après 15 minutes d'agitation à 65°C, 2,4 g d'iodure de potassium anhydre et 4,4 g de 3-chloro-3-méthyl-1-butyne sont ajoutés et le milieu réactionnel est maintenu 24 heures à 65°C puis 1 heure 30 à 130°C. Après refroidissement, la solution est hydrolysée puis extraite au dichlorométhane. Les phases organiques rassemblées sont lavées à l'eau, puis par une solution de potasse 1M, séchées sur sulfate de sodium, puis évaporées. Après chromatographie sur gel de silice (cyclohexane/acétone : 90/10), 1,10 g du produit attendu sont isolés.
***Point de fusion** : 225°C*

***Stade C** : 6-Méthoxy-3,3,14-triméthyl-7,14-dihydro-3H-benzo[b]pyrano[3,2-h]acridin-7-one*

A une solution de 0,5 g de produit du stade B dans 20 ml de diméthylformamide anhydre, sont additonnés lentement, à 0°C sous atmosphère inerte, 0,16 g d'hydrure de sodium, puis après 15 minutes 0,65 ml de diméthylsulfate (6 équivalents). Après 1 heures, le milieu réactionnel est hydrolysé par de la glace puis extrait à l'acétate d'éthyle. Après lavage de la phase organique par une solution aqueuse de soude, celle-ci est séchée sur sulfate de sodium, puis évaporée sous vide. Une chromatographie sur gel de silice (cyclohexane/acétone : 98/2) permet d'isoler 0,42 g du produit attendu.
***Point de fusion** : 188°C*

***Stade D** : (±) cis-1,2-Dihydroxy-6-méthoxy-3,3,14-triméthyl-2,3,7,14-tétrahydro-1H-benzo[b]pyrano[3,2-h]acridin-7-one*

A une solution de 2 g du produit du stade C et de 0,9 g de 4-méthylmorpholine-N-oxyde monohydrate dans 40 ml d'un mélange t-butanol/tétrahydrofurane/eau (10/3/1) est additionné 2,5 % de tétroxyde d'osmium en solution dans 3,8 ml de 2-méthyl-2-propanol. Après 2 jours à température ambiante, 105 ml d'une solution saturée en $NaHSO_3$ sont ajoutés et le milieu réactionnel est maintenu 1 heure sous agitation, puis extrait au dichlorométhane. Les phases organiques rassemblées sont séchées sur sulfate de sodium et concentrées sous vide. Une chromatographie sur gel de silice (dichlorométhane/méthanol : 95/5) permet d'isoler 1,3 g du produit attendu.
***Point de fusion** : 194°C*

**PREPARATION B : (±)-*cis*-1,2-Dihydroxy-6-(diméthylaminoéthylamino)-3,3,14-triméthyl-2,3,7,14-tétrahydro-1*H*-benzo[*b*]pyrano[3,2-*h*] acridin-7-one**

***Stade A : 6-(Diméthylaminoéthylamino)-3,3,14-triméthyl-7,14-dihydro-3H-benzo[b] pyrano[3,2-h]acridin-7-one***

A 0,15 g du produit obtenu au stade C de la préparation A est additionné 4 ml de N,N-diméthyléthylènediamine. Après 5 jours de réaction à 70 °C sous atmosphère inerte, le milieu réactionnel est évaporé sous pression réduite. Le résidu obtenu est chromatographié sur gel de silice (cyclohexane/acétate d'éthyle : 80/20) permettant d'isoler le produit attendu.
***Point de fusion** : huile*
***Spectre de masse** : (DIC/$NH_3$) : m/z : 428 $(M+H)^+$*

***Stade B : (±)-cis-1,2-Dihydroxy-6-(diméthylaminoéthylamino)-3,3,14-triméthyl-2,3,7,14-tétrahydro-1H-benzo[b] pyrano[3,2-h]acridin-7-one***

On procède comme dans le stade D de la préparation A en utilisant comme substrat le produit obtenu au stade précédent.

**PREPARATION C : (±)-*cis*-1,2-Dihydroxy-6-(diéthylaminopropylamino)-3,3,14-triméthyl-2,3,7,14-tétrahydro-1*H*-benzo[*b*]pyrano[3,2-*h*] acridin-7-one**

On procède comme dans la préparation B, des stades A à B, en utilisant comme réactif au stade A, la N,N-diéthylpropyldiamine.

**PREPARATION D : *cis*-6-[(2-Morpholin-4-yl)éthylamino]-1,2-dihydroxy-3,3,14-triméthyl-2,3,7,14-tétrahydro-1*H*-benzo[*b*]pyrano[3,2-*h*] acridin-7-one**

On procède comme dans la préparation B, des stades A à B, en utilisant comme réactif au stade A, la 4-(2-aminoéthyl)morpholine.

**PREPARATION E : *cis*-10,11-Dichloro-1,2-dihydroxy-6-méthoxy-3,3,14-triméthyl-2,3,7,14-tétrahydro-1*H*-benzo[*b*]pyrano[3,2-*h*] acridin-7-one**

On procède comme dans la préparation A, des stades A à D, en utilisant comme substrat au stade A l'acide 3-amino-6,7-dichloro-2-naphtalène-carboxylique.

**PREPARATION F : *cis*-1,2-Dihydroxy-6,9,12-triméthoxy-3,3,14-triméthyl-2,3,7,14-tétrahydro-1*H*-benzo[*b*]pyrano[3,2-*h*] acridin-7-one**

On procède comme dans la préparation A, des stades A à D, en utilisant comme substrat au stade A l'acide 3-amino-5,8-diméthoxy-2-naphtalène-carboxylique.

**Préparation G : *cis*-1,2-Dihydroxy-6-méthoxy-3,3-diméthyl-2,3,7,14-tétrahydro-1*H*-benzo[*b*]pyrano[3,2-*h*]acridin-7-one**

***Stade A : Acide 3-[(7-méthoxy-2,2-diméthyl-2H-chromèn-5-yl)amino]-2-naphthoique***

Un mélange de 1,2 mmole de 5-amino-6-méthoxy-2,2-diméthylchromène, 1,2 mmol d'acide 2-bromo-3-benzoïque, 0,327 g d'acétate de potassium, et 12 mg d'acétate de cuivre est mis en suspension dans 8 ml de 2-propanol et 0,25 ml de triéthylamine puis chauffé à reflux pendant 24 heures. Le milieu réactionnel est ensuite concentré sous pression réduite, repris par un mélange $CH_2Cl_2$/HCl 1N, extrait au dichlorométhane, séché et évaporé. Une chromatographie sur gel de silice du résidu (cyclohexane/acétate d'éthyle : 1/1) permet d'isoler le produit attendu.
***Spectre de masse** (DIC/$NH_3$) : 376 $[M+H]^+$*

***Stade B : 6-Méthoxy-3,3-diméthyl-3,14-dihydro-7H-benzo[b]pyrano[3,2-h]acridin-7-one***

1,02 mmol du composé obtenu au stade A dans 14 ml de dichlorométhane est traité par 1 ml d'acide trifluoroacétique. Après 2 heures à température ambiante, le milieu réactionnel est évaporé. Le résidu est repris par un mélange de dichlorométhane et d'une solution saturée en $NaHCO_3$, extrait au dichlorométhane, lavé par une solution de soude à 10 % puis réextrait au dichlorométhane. Après traitement classique, une chromatographie de résidu sur gel de silice (dichlorométhane/méthanol : 98/2) permet d'isoler le produit attendu.
***Spectre de masse I.E.** : m/z : 357 ($M^+$); 342 $(M-15)^+$*

***Stade C : cis-1,2-dihydroxy-6-méthoxy-3,3-diméthyl-2,3,7,14-tétrahydro-1H-benzo[b] pyrano[3,2-h]acridin-7-one***

Un mélange de 0,279 mmol du composé obtenu au stade B, d'une solution de tétraoxyde d'osmium à 2,5 % dans 3,8 ml de 2-méthyl-2-propanol et de 60 mg de N-oxyde de 4-méthylmorpholine est mis en solution dans 5 ml d'un mélange 10/3/1 de tBuOH/tétrahydrofurane/$H_2O$. Après 24 heures d'agitation à température ambiante, 5 ml d'une solution saturée de $NaHSO_3$ est ajoutée. Après 1 heure d'agitation, le milieu réactionnel est extrait au dichlorométhane. La phase organique est ensuite séchée puis concentrée sous pression réduite. Une chromatographie sur gel de silice du résidu (dichlorométhane/méthanol: 97/3) permet d'isoler le produit attendu.
***Spectre de masse** (DIC/$NH_3$) : 392 $[M+H]^+$*

**EXEMPLE 1 : Acide (±)-cis-4-{[1-(acétyloxy)-6-méthoxy-3,3,14-triméthyl-7-oxo -2,3,7,14-tétrahydro-1*H*-benzo [*b*]pyrano[3,2-*h*]acridin-2-yl]oxy}-4-oxobutanoïque**

A une solution de 0,74 mmol du composé de la préparation A dans 7 ml de pyridine anhydre sont ajoutés 5 équivalents d'anhydride succinique et 1 mg de diméthylaminopyridine. Après 17 heures d'agitation à température ambiante et dans l'obscurité, 25 ml d'anhydride acétique sont ajoutés. Le milieu réactionnel est refroidi à -15°C et l'agitation est maintenue 1 heure 30. Le milieu réactionnel est ensuite concentré sous pression réduite. Une chromatographie sur gel de silice (dichlorométhane/méthanol) permet d'isoler le produit attendu.
***Spectre de masse : (FAB) : m/z = 548 [M+H]$^+$***
***Point de fusion : 154°C***

**EXEMPLE 2 : Acide (±)-*cis*-4-{[1-(acétyloxy)-6-{[2-(diméthylamino)éthyl]amino}-3,3,14-triméthyl-7-oxo-2,3,7,14-tétrahydro-1*H*-benzo[*b*]pyrano [3,2-*h*]acridin-2-yl)oxy]-4-oxobutanoïque**

On procède comme dans l'exemple 1 en utilisant comme substrat le composé de la préparation B.

**EXEMPLE 3 : Acide (±)-*cis*-5-{[1-(acétyloxy)-6-méthoxy-3,3,14-triméthyl-7-oxo-2,3,7,14-tétrahydro-1*H*-benzo [*b*]pyrano[3,2-*h*]acridin-2-yl]oxy}-5-oxopentanoïque**

On procède comme dans l'exemple 1 en utilisant comme réactif l'anhydride glutarique à la place de l'anhydride succinique.
***Spectre de masse : (DIC/$NH_3$) : m/z = 562 [M+H]$^+$***
***Point de fusion : 155°C***

**EXEMPLE 4 : (±)-*cis*-1-(Acétyloxy)-6-méthoxy-3,3,14-triméthyl-7-oxo-2,3,7,14-tétrahydro-1*H*-benzo[*b*]pyrano [3,2-*h*]acridin-2-yl-2,3-dihydroxypropanoate**

***Stade A : (±)-cis-1-(Acétyloxy)-6-méthoxy-3,3,14-triméthyl-7-oxo-2,3,7,14-tétrahydro-1H-benzo[b]pyrano [3,2-h]acridin-2-yl acrylate***

A une solution de 0,5 mmol du composé de la préparation A dans 3 ml de pyridine anhydre est ajouté 0,6 mmol de chlorure d'acryloyle. Après 2,5 jours d'agitation à température ambiante et dans l'obscurité, 2 ml d'anhydride acétique sont additionnés et l'agitation est maintenue pendant 48 heures. Le milieu réactionnel est ensuite concentré sous pression réduite. Une chromatographie sur gel de silice (dichlorométhane) permet d'isoler le produit attendu.
***Spectre de masse : (DIC/$NH_3$) : m/z = 502 [M+H]$^+$***

***Stade B: (±)-cis-1-(Acétyloxy)-6-méthoxy-3,3,14-triméthyl-7-oxo-2,3,7,14-tétrahydro-1H-benzo[b]pyrano[3,2-h] acridin-2-yl-2,3-dihydroxypropanoate***

A une suspension de 0,4 mmol du composé obtenu dans le stade A dans 5 ml d'un mélange *tert*-butanol/tétrahydrofurane/eau (10/3/1 volume à volume), sont ajoutés 1 ml de solution de trétroxyde d'osmium à 2,5 % dans le tert-butanol et 4,4 mmol de 4-méthylmorpholine-N-oxyde monohydraté. Après 2 jours d'agitation à température ambiante, 50 ml d'une solution aqueuse saturée de $NaHCO_3$ sont additionnés. Après une heure d'agitation, extraction au dichlorométhane, et concentration sous pression réduite, une chromatographie sur gel de silice (dichlorométhane/méthanol : 96/4) permet d'isoler le produit attendu.
***Spectre de masse : (DIC/$NH_3$) : m/z = 536 [M+H]$^+$***

**EXEMPLE 5 : (±)-*cis*-1-(Acétyloxy)-6-méthoxy-3,3,14-triméthyl-7-oxo-2,3,7,14-tétrahydro-1*H*-benzo[*b*]pyrano [3,2-*h*]acridin-2-yl-2-[(*tert*-butoxycarbonyl)amino]acétate**

A une solution de 0,5 mmol du composé de la Préparation A et 0,5 mmol d'acide 2-[(*tert*-butoxycarbonyl) amino]acétique dans 10 ml de diméthylformamide, refroidie à 0°C, est ajouté lentement 0,6 mmol de dicyclohexylcarbodiimide. Le milieu réactionnel est maintenu 5 heures à 0°C puis 16 heures à température ambiante. Après filtration et évaporation sous pression réduite, le résidu est mis en solution dans 2 ml de pyridine anhydre, additionné de 2 ml d'anhydride acétique, et agité à température ambiante et à l'obscurité pendant 48 heures. Après concentration sous pression réduite du milieu réactionnel, une chromatographie sur gel de silice du résidu (dichlorométhane) permet d'isoler le produit attendu.
***Spectre de masse : (DIC/$NH_3$) : m/z = 605 [M+H]$^+$***

**EXEMPLE 6 : (±)-*cis*-1-(Acétyloxy)-6-méthoxy-3,3,14-triméthyl-7-oxo-2,3,7,14-tétrahydro-1*H*-benzo[*b*]pyrano [3,2-*h*]acridin-2-yl-2-aminoacétate**

A une solution de 0,1 mmol du composé de l'exemple 5 dans 1 ml de chloroforme est additionné à température ambiante 0,14 µl d'iodotriméthylsilane. Le milieu réactionnel est agité 5 minutes à température ambiante puis évaporé à sec sous pression réduite. Une chromatographie sur gel de silice (dichlorométhane/méthanol : 85/15) permet d'isoler le produit attendu.
***Spectre de masse :** (DIC/$NH_3$) : m/z = 505 $[M+H]^+$*

**Exemple 7 : Acide (±)-*cis*-4-({1-[(3-carboxypropanoyl)oxy]-6-méthoxy-3,3,14-triméthyl-7-oxo-2,3,7,14-tétrahydro-1*H*-benzo[*b*]pyrano[3,2-*h*] acridin-2-yl}oxy)-4-oxobutanoïque**

Le produit est isolé lors de la chromatographie du composé de l'exemple 1.
***Spectre de Masse** (FAB) : m/z : 606 $[M+H]^+$*
***Point de fusion** : 128°C*

**Exemple 8 : Acide *cis*-4-{[1-(acétyloxy)-10,11-dichloro-6-méthoxy-3,3,14-triméthyl-7-oxo-2,3,7,14-tétrahydro-1*H*-benzo[*b*]pyrano[3,2-*h*] acridin-2-yl]oxy}-4-oxobutanoïque**

On procède comme dans l'exemple 1 en utilisant comme substrat le composé de la préparation E.

**Exemple 9 : Acide *cis*-4-{[1-(acétyloxy)-6,9,12-triméthoxy-3,3,14-triméthyl-7-oxo-2,3,7,14-tétrahydro-1*H*-benzo [b]pyrano[3,2-*h*]acridin-2-yl]oxy}-4-oxobutanoïque**

On procède comme dans l'exemple 1 en utilisant comme substrat le composé de la préparation F.

**Exemple 10 : Acide *cis*-4-[(1-(acétyloxy)-6-{[3-(diéthylamino)propyl]amino}-3,3,14-triméthyl-7-oxo-2,3,7,14-tétrahydro-1*H*-benzo[*b*]pyrano[3,2-*h*] acridin-2-yl)oxy]-4-oxobutanoïque**

On procède comme dans l'exemple 1 en utilisant comme substrat le composé de la préparation C.

**Exemple 11 : Acide *cis*-4-[(1-(acétyloxy)-3,3,14-triméthyl-6-{[2-(4-morpholinyl) éthyl]amino}-7-oxo-2,3,7,14-tétrahydro-1*H*-benzo[*b*]pyrano[3,2-*h*] acridin-2-yl)oxy]-4-oxobutanoïque**

On procède comme dans l'exemple 1 en utilisant comme substrat le composé de la préparation D.

**Exemple 12 : Acide *cis*-4-{[6-méthoxy-3,3,14-triméthyl-7-oxo-1-(propionyloxy)-2,3,7,14-tétrahydro-1*H*-benzo[*b*] pyrano[3,2-*h*]acridin-2-yl]oxy}-4-oxobutanoïque**

On procède comme dans l'exemple 1 en utilisant comme réactif l'anhydride propionique à la place de l'anhydride acétique.

**Exemple 13 : Acide *cis*-4-{[1-(isobutyryloxy)-6-méthoxy-3,3,14-triméthyl-7-oxo-2,3,7,14-tétrahydro-1*H*-benzo [*b*]pyrano[3,2-*h*]acridin-2-yl]oxy}-4-oxobutanoïque**

On procède comme dans l'exemple 1 en utilisant comme réactif l'anhydride isobutyrique à la place de l'anhydride acétique.

**Exemple 14 : Acide *cis*-4-{[1-(benzoyloxy)-6-méthoxy-3,3,14-triméthyl-7-oxo-2,3,7,14-tétrahydro-1*H*-benzo[*b*] pyrano[3,2-*h*]acridin-2-yl]oxy}-4-oxobutanoïque**

On procède comme dans l'exemple 1 en utilisant comme réactif l'anhydride benzoïque à la place de l'anhydride acétique.

**Exemple 15 : Acide *cis*-4-{[6-méthoxy-3,3,14-triméthyl-7-oxo-1-(pentanoyloxy)-2,3,7,14-tétrahydro-1*H*-benzo [*b*]pyrano[3,2-*h*]acridin-2-yl]oxy}-4-oxobutanoïque**

On procède comme dans l'exemple 1 en utilisant comme réactif l'anhydride valérique à la place de l'anhydride

acétique.

**Exemple 16 : *cis*-[1-(acétyloxy)-6-méthoxy-3,3,14-triméthyl-7-oxo-2,3,7,14-tétrahydro-1*H*-benzo[*b*]pyrano [3,2-*h*]acridin-2-yl] (diméthylamino)acétate**

300 mg du composé de la préparation A est mis en solution dans 4 mL de diméthyl formamide anhydre à 0°C. Le milieu est additionné de 90 mg de 4-diméthylaminopyridine et de 152 mg de *N,N*-diméthylglycine. Après un repos de 5 minutes à 0°C, on ajoute 142 mg de chlorhydrate de 1-(3-diméthylaminopropyl)-3-éthylcarbodiimide. Le milieu réactionnel est agité pendant 4 heures puis additionné de 2 ml d'eau glacée. Le milieu est extrait par du dichlorométhane. Les solutions organiques sont réunies, séchées sur sulfate de sodium anhydre, filtrées puis distillées sous pression réduite. Le résidu obtenu foumit, après chromatographie sur gel de silice ($CH_2Cl$/MeOH : 85/15) le produit attendu.
***Spectre de masse** (DIC/$NH_3$) : 533 $[M+H]^+$*

**Exemple 17 : *cis*-[1-(acétyloxy)-6-méthoxy-3,3,14-triméthyl-7-oxo-2,3,7,14-tétrahydro-1*H*-benzo[*b*]pyrano [3,2-*h*]acridin-2-yl]-4-(diméthylamino)butanoate**

On procède comme dans l'exemple 16 en utilisant comme réactif l'acide 4-diméthylamino-butyrique à la place de la N,N-diméthylglycine.

**Exemple 18 : *cis*-[1-(acétyloxy)-6-méthoxy-3,3,14-triméthyl-7-oxo-2,3,7,14-tétrahydro-1*H*-benzo[*b*]pyrano [3,2-*h*]acridin-2-yl] (acétylamino)acétate**

On procède comme dans l'exemple 16 en utilisant comme réactif la N-acétylglycine à la place de la N,N-diméthylglycine.
***Spectre de Masse** (DIC/$NH_3$) : 547 $[M+H]^+$*

**Exemple 19 : Acide *cis*-4-{[1-(acétyloxy)-6-méthoxy-3,3-diméthyl-7-oxo-2,3,7,14-tétrahydro-1*H*-benzo[*b*]pyrano[3,2-*h*]acridin-2-yl]oxy}-4-oxobutanoique**

On procède comme dans l'exemple 1 en utilisant comme substrat le composé de la préparation G.

**Exemple 20 : sel de lysine d'Acide (±) *cis*-4-{[1-(acétyloxy)-6-méthoxy-3,3,14-triméthyl-7-oxo-2,3,7,14-tétrahydro-1*H*-benzo[*b*]pyrano[3,2-*h*] acridin-2-yl]oxy}-4-oxobutanoique**

0,1 g du composé de l'exemple 1 est dissout dans 20 ml d'éthanol en présence d'un équivalent d'hydrate de lysine. La solution est agitée pendant 30 minutes puis évaporée et concentrée sous pression réduite permettant d'isoler le produit attendu sous forme de sel.

**Exemple 21 : sel de lysine d' Acide (±)-*cis*-4-({1-[(3-carboxypropanoyl)oxy]-6-méthoxy-3,3,14-triméthyl-7-oxo-2,3,7,14-tétrahydro-1*H*-benzo[*b*] pyrano[3,2-*h*]acridin-2-yl}oxy)-4-oxobutanoïque**

On procède comme dans l'exemple 20 en utilisant comme substrat le composé de l'exemple 7 et 2 équivalents de lysine.

**Exemple 22 : Acide *cis*-5-({1-[(4-carboxybutanoyl)oxy]-6-méthoxy-3,3,14-triméthyl-7-oxo-2,3,7,14-tétrahydro-1*H*-benzo[*b*]pyrano[3,2-*h*] acridin-2-yl}oxy)-5-oxopentanoique**

Le produit est isolé lors de la chromatographie du composé de l'exemple 3.
***Spectre de Masse** (DIC/$NH_3$) : 634 $[M+H]^+$*
***Point de fusion** : 118°C*

**Exemple 23 : Acide *cis*-5-{[1-(acétyloxy)-6-méthoxy-3,3,14-triméthyl-7-oxo-2,3,7,14-tétrahydro-1*H*-benzo[*b*]pyrano[3,2-*h*]acridin-2-yl]oxy}-2,4-diméthyl-5-oxopentanoique**

On procède comme dans l'exemple 1 en utilisant comme réactif l'acide 2,4-diméthylglutarique à la place de l'anhydride succinique.

**Exemple 24 : Acide *cis*-5-{[1-(acétyloxy)-6-méthoxy-3,3,14-triméthyl-7-oxo-2,3,7,14-tétrahydro-1*H*-benzo[*b*]pyrano[3,2-*h*]acridin-2-yl]oxy}-3-hydroxy-3-méthyl-5-oxopentanoique**

On procède comme d ans l'exemple 1 en utilisant comme réactif l'acide 3-hydroxy-3-méthyl-glutarique à la place de l'anhydride succinique.

**Exemple 25 : *cis*-1-(acétyloxy)-6-méthoxy-3,3,14-triméthyl-7-oxo-2,3,7,14-tétrahydro-1*H*-benzo[*b*]pyrano [3,2-*h*]acridin-2-yl 5-(benzylamino)-5-oxopentanoate**

On procède comme d ans l'exemple 1 en utilisant comme réactif l'acide benzylglutaramique à la place de l'anhydride succinique.

**Exemple 26 : *cis*-1-(acétyloxy)-6-méthoxy-3,3,14-triméthyl-7-oxo-2,3,7,14-tétrahydro-1*H*-benzo[*b*]pyrano [3,2-*h*]acridin-2-yl 5-(4-méthoxyphényl)-5-oxopentanoate**

On procède comme dans l'exemple 1 en utilisant comme réactif l'acide 5-(4-méthoxyphényl)-5-oxovalérique à la place de l'anhydride succinique.

***ETUDE PHARMACOLOGIQUE DES COMPOSES DE L'INVENTION***

**EXEMPLE 27 : Activité in vitro**

La leucémie murine L1210 et le carcinome du colon humain HT-29 ont été utilisés in vitro. Les cellules sont cultivées dans du milieu de culture RPMI 1640 complet contenant 10 % de sérum de veau foetal, 2 mM de glutamine, 50 U/ml de pénicilline, 50 μg/ml de streptomycine et 10 mM d'Hepes, pH : 7,4. Les cellules sont réparties dans des microplaques et exposées aux composés cytotoxiques pendant 4 temps de doublement, soit 48 heures (L1210) ou 96 heures (HT-29). Le nombre de cellules viables est ensuite quantifié par un essai colorimétrique, le Microculture Tetrazolium Assay (J. Carmichael et al., Cancer Res., 47, 936-942, (1987)). Les résultats sont exprimés en $IC_{50}$, concentration en cytotoxique qui inhibe à 50 % la prolifération des cellules traitées.
A titre d'exemple, les composés des exemples 1 et 7 montrent respectivement un $IC_{50}$ de 2 μM et 2,3 μM, démontrant ainsi leur plus grande activité que le composé de référence, l'acronycine.

**EXEMPLE 28 : Activité in vivo**

***1- Activité antitumorale sur la leucémie P 388***

La lignée P 388 (leucémie mutine) a été fournie par le National Cancer Institute (Frederick, USA). Les cellules tumorales ($10^6$ cellules) ont été inoculées au jour 0 dans la cavité péritonéale de souris B6D2F1 femelles (Iffa Credo, France). Six souris de 18 à 20 g ont été utilisées par groupe expérimental. Les produits ont été administrés par voie intrapéritonéale au jour 1.
L'activité antitumorale est exprimée en % de T/C:

$$\text{T/C \% (surve)} = \frac{\text{Temps de survie médian des animaux traités}}{\text{Temps de survie médian des animaux contrôles}} \times 100$$

Les composés de l'invention sont très actifs dans ce modèle, alors que l'acronycine n'est que marginalement active, et induisent des T/C > 150 % à des doses inférieures à 100 mg/kg.

***2-Activité antitumorale sur l'adénocarcinome du colon C38***

Les fragments tumoraux d'adénocarcinome du colon C38, pesant environ 30 mg, ont été implantés au jour 0 sous la peau de souris B6D2F1 (Iffa Credo, France).
Après croissance de la tumeur, les souris ont été séparées en groupes contrôle (18 animaux) et traité (6 à 7 animaux), homogènes quant à la taille tumorale . Les produits ont été administrés par voie i.v. une fois par semaine pendant 3 semaines (aux jours 10, 17 et 24), à leur Dose Maximale Tolérée (MTD), MTD/2 et MTD/4.
Les tumeurs ont été mesurées deux fois par semaine et les volumes tumoraux ont été calculés suivant la formule : Volume ($mm^3$) = longueur (mm) x largeur ($mm^2$) /2.
L'activité antitumorale est exprimée en % de T/C :

$$\% \ T/C = \frac{\text{Vt/V0 médian des animaux traités}}{\text{Vt/V0 médian des animaux contrôle}} \times 100$$

V0 et Vt étant respectivement le volume initial de la tumeur et son volume au temps de mesure t.

La dose optimale est la dose qui donne le T/C le plus bas sans toxicité (mort prématurée ou perte de poids supérieure à 20%).

Les composés de l'invention se sont révélés plus actifs, dans ce modèle, que le composé de référence constitué par l'acronycine permettant de démontrer leur fort potentiel thérapeutique.

**EXEMPLE 29 : Composition pharmaceutique : soluté injectable**

| | |
|---|---|
| Composé de l'exemple 1 | 10 mg |
| Eau distillée pour préparations injectables | 25 ml |

## Revendications

**1.** Composés de formule (I) :

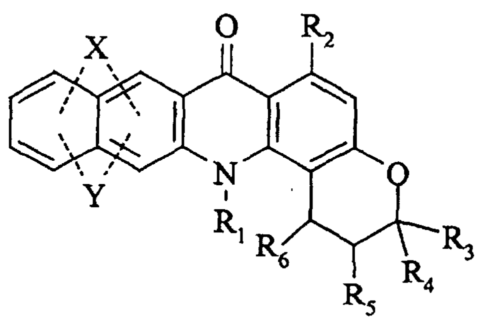

(I)

dans laquelle :

X, Y, identiques ou différents, indépendamment l'un de l'autre, représentent un groupement choisi parmi atome d'hydrogène, d'halogène, groupement hydroxy, mercapto, cyano, nitro, alkyle ($C_1$-$C_6$) linéaire ou ramifié, alkoxy ($C_1$-$C_6$) linéaire ou ramifié, trihalogénoalkyle ($C_1$-$C_6$) linéaire ou ramifié, amino éventuellement substitué par un ou deux groupements, identiques ou différents, alkyle ($C_1$-$C_6$) linéaires ou ramifiés eux-mêmes éventuellement substitués par un groupement alkoxy ($C_1$-$C_6$) linéaire ou ramifié ou par un groupement de formule $-NR_7R_8$ dans laquelle $R_7$ et $R_8$, identiques ou différents, indépendamment l'un de l'autre, représentent un atome d'hydrogène, un groupement alkyle ($C_1$-$C_6$) linéaire ou ramifié, aryle ou arylalkyle ($C_1$-$C_6$) linéaire ou ramifié, ou forment ensemble un groupement méthylènedioxy ou un groupement éthylènedioxy, étant entendu que les substituants X et Y peuvent être présents sur l'un ou l'autre des deux cycles benzéniques adjacents,

$R_1$ représente un atome d'hydrogène ou un groupement alkyle ($C_1$-$C_6$) linéaire ou ramifié,

$R_2$ représente :

- un atome d'hydrogène,
- un groupement hydroxy,
- un groupement alkyle ($C_1$-$C_6$) linéaire ou ramifié,
- un groupement alkoxy ($C_1$-$C_6$) linéaire ou ramifié, éventuellement substitué par un groupement choisi parmi :

  * groupement de formule $NR_9R_{10}$ dans laquelle $R_9$ et $R_{10}$, identiques ou différents, indépen-

damment l'un de l'autre, représentent un atome d'hydrogène, un groupement alkyle ($C_1$-$C_6$) linéaire ou ramifié, ou un groupement hydroxyalkyle ($C_1$-$C_6$) linéaire ou ramifié,
* et hétérocycle saturé ou insaturé, monocyclique ou bicyclique, de 5 à 7 chaînons comportant un ou deux hétéroatomes choisis parmi oxygène, azote et soufre,

- un groupement alkylcarbonyloxy ($C_1$-$C_6$) linéaire ou ramifié,
- ou un groupement amino éventuellement substitué :

  * par un ou deux groupements, identiques ou différents, alkyle ($C_1$-$C_6$) linéaire ou ramifié, aryle, ou arylalkyle ($C_1$-$C_6$) linéaire ou ramifié,
  * par un groupement alkylcarbonyle ($C_1$-$C_6$) linéaire ou ramifié, éventuellement substitué par un groupement $-NR_7R_8$ avec $R_7$ et $R_8$ tels que définis précédemment,
  * par un groupement de formule $-R_{11}-NR_9R_{10}$, dans laquelle $R_{11}$ représente un groupement alkylène ($C_1$-$C_6$) linéaire ou ramifié et $R_9$, $R_{10}$, identiques ou différents, indépendamment l'un de l'autre représentent un atome d'hydrogène, un groupement alkyle ($C_1$-$C_6$) linéaire ou ramifié, ou un groupement hydroxyalkyle ($C_1$-$C_6$) linéaire ou ramifié,
  * par un groupement alkylène ($C_1$-$C_6$) linéaire ou ramifié, substitué par un hétérocycle saturé ou insaturé, monocyclique ou bicyclique, de 5 à 7 chaînons comportant un ou deux hétéroatomes choisis parmi oxygène, azote et soufre,
  * ou par un groupement de formule $-R_{11}-CO-R_{12}$ dans laquelle $R_{11}$ est tel que défini précédemment, et $R_{12}$ représente un groupement hydroxy ou alkoxy ($C_1$-$C_6$) linéaire ou ramifié,

$R_3$, $R_4$, identiques ou différents, indépendamment l'un de l'autre, représentent un atome d'hydrogène ou un groupement alkyle ($C_1$-$C_6$) linéaire ou ramifié,

$R_5$ et/ou $R_6$ représente(nt) un groupement de formule -O-CO-U-V dans laquelle :

- U représente une chaîne alkylène ($C_1$-$C_8$) linéaire ou ramifié, éventuellement substituée par un ou plusieurs groupements, identiques ou différents, choisis parmi aryle, hydroxy et alkoxy ($C_1$-$C_6$) linéaire ou ramifié,
- V représente un groupement choisi parmi :

  * carboxy,
  * $-CO_2R_{13}$ dans lequel $R_{13}$ représente un groupement alkyle ($C_1$-$C_6$) linéaire ou ramifié éventuellement substitué par un ou plusieurs groupements hydroxy, aryle ou arylalkyle ($C_1$-$C_6$) linéaire ou ramifié,
  * hydroxy,
  * alkoxy ($C_1$-$C_6$) linéaire ou ramifié,
  * $-NR_7R_8$ dans lequel $R_7$ et $R_8$, identiques ou différents, sont tels que définis précédemment,
  * $-NR_7-CO_2R_{13}$ dans lequel $R_7$ et $R_{13}$ sont tels que définis précédemment,
  * $-NR_7-COR_{13}$ dans lequel $R_7$ et $R_{13}$ sont tels que définis précédemment,
  * $-COR_{13}$ dans lequel $R_{13}$ est tel que défini précédemment,
  * et $-CO-NR_7R_8$ dans lequel $R_7$ et $R_8$, identiques ou différents, sont tels que définis précédemment,

et dans le cas où un seul des deux groupements $R_5$ et $R_6$ représente un groupement de formule -O-CO-U-V, alors l'autre desdits groupements $R_5$ ou $R_6$ représente un groupement Z choisi parmi :

- hydroxy,
- alkoxy ($C_1$-$C_6$) linéaire ou ramifié,
- alkylcarbonyloxy ($C_1$-$C_6$) linéaire ou ramifié,
- arylcarbonyloxy,
- arylalkylcarbonyloxy ($C_1$-$C_6$) linéaire ou ramifié,
- et amino éventuellement substitué par un ou deux groupements alkyle ($C_1$-$C_6$) linéaires ou ramifiés, identiques ou différents,

leurs isomères optiques, N-oxydes, ainsi que leurs sels d'addition à un acide ou à une base pharmaceutiquement acceptable,

étant entendu que par "aryle", on comprend un groupement phényle ou naphtyle, comportant éventuellement une ou plusieurs substitutions, identiques ou différentes, choisies parmi hydroxy, halogéno, carboxy, nitro, amino, alkylamino ou dialkylamino ($C_1$-$C_6$) linéaire ou ramifié, alkoxy ($C_1$-$C_6$) linéaire ou ramifié, acyle ($C_1$-$C_6$) linéaire ou ramifié, et alkylcarbonyloxy ($C_1$-$C_6$) linéaire ou ramifié.

**2.** Composés de formule (I) selon la revendication 1 **caractérisés en ce que** $R_3$ et $R_4$, identiques ou différents, représentent un groupement alkyle ($C_1$-$C_6$) linéaire ou ramifié, leurs isomères optiques, N-oxydes, ainsi que leurs sels d'addition à un acide ou à une base pharmaceutiquement acceptable.

**3.** Composés de formule (I) selon l'une quelconque des revendications 1 ou 2, **caractérisés en ce que** $R_3$ et $R_4$, identiques, représentent chacun un groupement méthyle, leurs isomères optiques, N-oxydes, ainsi que leurs sels d'addition à un acide ou à une base pharmaceutiquement acceptable.

**4.** Composés de formule (I) selon la revendication 1 **caractérisés en ce que** $R_2$ représente un groupement alkoxy ($C_1$-$C_6$) linéaire ou ramifié, ou un groupement amino éventuellement substitué tel que défini dans la formule (I), leurs isomères optiques, N-oxydes, ainsi que leurs sels d'addition à un acide ou à une base pharmaceutiquement acceptable.

**5.** Composés de formule (I) selon la revendication 1 **caractérisés en ce que** $R_5$ représente un groupement de formule -O-CO-U-V dans laquelle U et V sont tels que définis dans la formule (I) et $R_6$ représente un groupement Z tel que défini dans la formule (I), leurs isomères optiques, N-oxydes, ainsi que leurs sels d'addition à un acide ou à une base pharmaceutiquement acceptable.

**6.** Composés de formule (I) selon la revendication 1 **caractérisés en ce que** $R_6$ et $R_5$, identiques, représentent chacun un groupement de formule -O-CO-U-V dans laquelle U et V sont tels que définis dans la formule (I), leurs isomères optiques, N-oxydes, ainsi que leurs sels d'addition à un acide ou à une base pharmaceutiquement acceptable.

**7.** Composés de formule (I) selon l'une quelconque des revendications 1 ou 5 **caractérisés en ce que** $R_5$ représente un groupement de formule -O-CO-U-V dans laquelle U représente une chaîne alkylène ($C_1$-$C_4$) linéaire et V représente un groupement choisi parmi carboxy, - $NR_7R_8$, -$NR_7$-$CO_2R_{13}$ et -$NR_7$-$COR_{13}$ dans lesquels $R_7$, $R_8$ et $R_{13}$, identiques ou différents, représentent un atome d'hydrogène ou un groupement alkyle ($C_1$-$C_6$) linéaire ou ramifié, leurs isomères optiques, N-oxydes, ainsi que leurs sels d'addition à un acide ou à une base pharmaceutiquement acceptable.

**8.** Composés de formule (I) selon la revendication 1 **caractérisés en ce que** $R_6$ représente un groupement alkylcarbonyloxy ($C_1$-$C_6$) linéaire ou ramifié, leurs isomères optiques, N-oxydes, ainsi que leurs sels d'addition à un acide ou à une base pharmaceutiquement acceptable.

**9.** Composés de formule (I) selon la revendication 1 qui sont le :

- acide (+)-cis-4-{[1-(acétyloxy)-6-méthoxy-3,3,14-triméthyl-7-oxo-2,3,7,14-tétrahydro-1*H*-benzo[b]pyrano [3,2-h]acridin-2-yl]oxy}-4-oxobutanoïque,
- acide (±)-*cis*-4-({1-[(3-carboxypropanoyl)oxy]-6-méthoxy-3,3,14-triméthyl-7-oxo-2,3,7,14-tétrahydro-1*H*-benzo[*b*]pyrano[3,2-*h*]acridin-2-yl}oxy)-4-oxobutanoïque,
- acide (±)-cis-5-{[1-(acétyloxy)-6-méthoxy-3,3,14-triméthyl-7-oxo-2,3,7,14-tétrahydro-1*H*-benzo[b]pyrano [3,2-h]acridin-2-yl]oxy}-5-oxopentanoïque,
- *cis*-[1-(acétyloxy)-6-méthoxy-3,3,14-triméthyl-7-oxo-2,3,7,14-tétrahydro-1*H*-benzo[*b*] pyrano[3,2-*h*]acridin-2-yl](diméthylamino)acétate,
- et *cis*-[1-(acétyloxy)-6-méthoxy-3,3,14-triméthyl-7-oxo-2,3,7,14-tétrahydro-1*H*-benzo[*b*] pyrano[3,2-*h*]acridin-2-yl ]-4-(diméthylamino)butanoate,

leurs isomères optiques, N-oxydes, ainsi que leurs sels d'addition à un acide ou à une base pharmaceutiquement acceptable.

**10.** Procédé de préparation des composés de formule (I) selon la revendication 1 **caractérisé en ce que** l'on fait réagir :

- *soit un dérivé de l'acide 3-amino-2-naphtalène carboxylique (II) :*

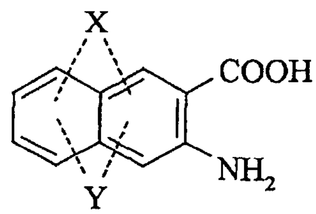

(II)

dans laquelle X et Y sont tels que définis dans la formule (I), avec un dérivé du phloroglucinol de formule (III) :

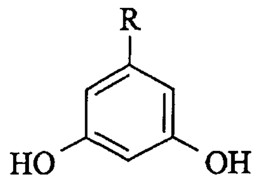

(III)

dans laquelle R représente un atome d'hydrogène, un groupement hydroxy ou un groupement alkyle ($C_1$-$C_6$) linéaire ou ramifié,
pour conduire aux composés de formule (IV) :

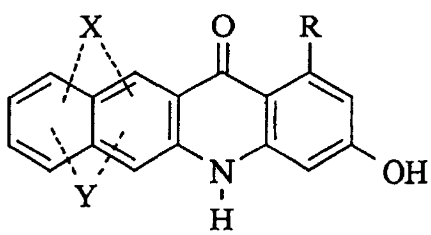

(IV)

dans laquelle X, Y et R sont tels que définis précédemment,
qui sont ensuite traités en condition basique dans un solvant aprotique par un alcyne de formule (V) :

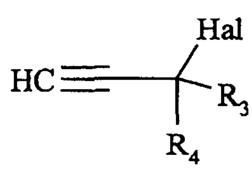

(V)

dans laquelle Hal représente un atome d'halogène et $R_3$ et $R_4$ sont tels que définis dans la formule (I),
pour conduire aux composés de formule (VI) :

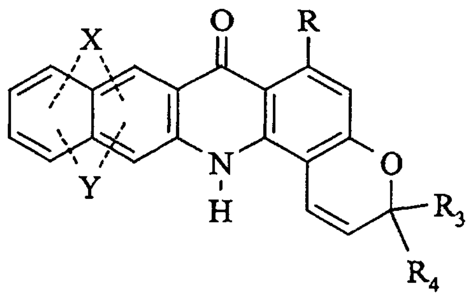

(VI)

dans laquelle X, Y, R, $R_3$ et $R_4$ sont tels que définis précédemment,

- *soit un dérivé de l'acide 3-halogéno-2-naphtalène carboxylique de formule (VII) :*

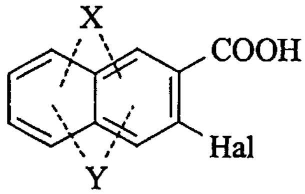

**(VII)**

dans laquelle X et Y sont tels que définis dans la formule (I) et Hal représente un atome d'halogène, avec un dérivé amino-chromène de formule (VIII) :

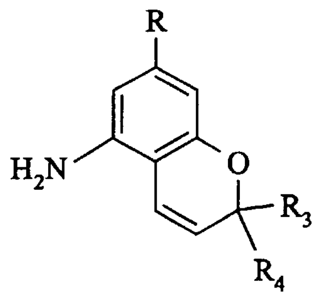

**(VIII)**

dans laquelle $R_3$ et $R_4$ sont tels que définis dans la formule (I) et R a la même signification que précédemment, pour conduire également aux composés de formule (VI) :

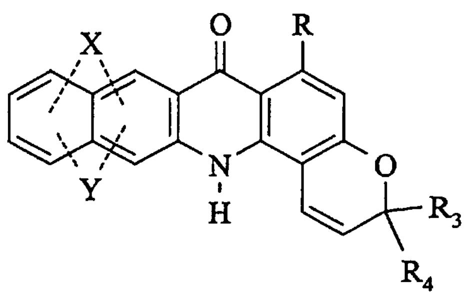

**(VI)**

dans laquelle X, Y, R, $R_3$ et $R_4$ sont tels que définis précédemment,
composés de formule (VI) dont l'atome d'azote est éventuellement substitué, par action d'un halogénure d'alkyle ou d'un sulfate de dialkyle en présence d'un agent de déprotonation, en solvant polaire aprotique, permettant d'obtenir les composés de formule (IX) :

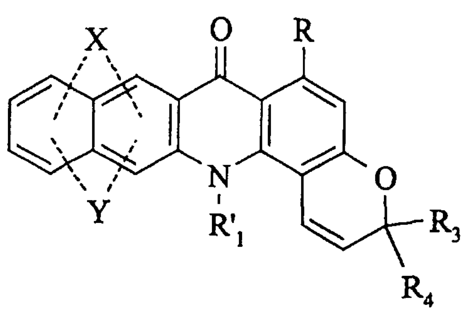

**(IX)**

dans laquelle X, Y, R, $R_3$ et $R_4$ sont tels que définis précédemment, et $R'_1$ représente un groupement alkyl ($C_1$-$C_6$) linéaire ou ramifié,

composés de formule (IX) qui sont soumis à l'action d'un agent alkylant tel qu'un sulfate de dialkyle, ou d'un agent acylant, pour conduire aux composés de formule (X) :

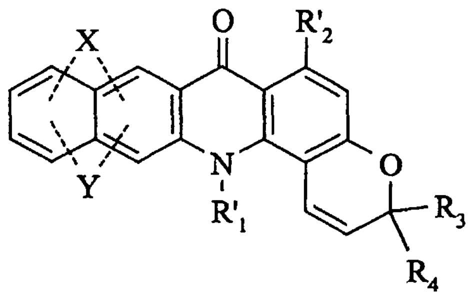

**(X)**

dans laquelle X, Y, R'$_1$, $R_3$ et $R_4$ sont tels que définis précédemment et R'$_2$ représente un groupement alkoxy (éventuellement substitué par un groupement de formule $NR_9R_{10}$ dans laquelle $R_9$ et $R_{10}$ sont tels que définis dans la formule (I)), ou alkylcarbonyloxy ($C_1$-$C_6$) linéaire ou ramifié,
composé de formule (X) qui est traité, dans le cas où R'$_2$ représente un groupement alkoxy par exemple, par un composé de formule (XI) :

HNRaRb **(XI)**

dans laquelle Ra représente un atome d'hydrogène, un groupement alkyle ($C_1$-$C_6$) linéaire ou ramifié, aryle, ou arylalkyle ($C_1$-$C_6$) linéaire ou ramifié, et Rb représente un atome d'hydrogène, un groupement alkyle ($C_1$-$C_6$) linéaire ou ramifié, aryle, arylalkyle ($C_1$-$C_6$) linéaire ou ramifié, un groupement de formule $-R_{11}$-$NR_9R_{10}$ dans laquelle $R_{11}$, $R_{10}$ et $R_9$ sont tels que définis dans la formule (I), un groupement alkylcarbonyle ($C_1$-$C_6$) linéaire ou ramifié la partie alkyle étant éventuellement substituée par un groupement $NR_7R_8$ tel que défini dans la formule (I), un groupement hétérocycloalkylène (les termes alkylène et hétérocycle ayant la même signification que dans la formule (I)) ou un groupement de formule $-R_{11}$-CO-$R_{12}$ dans laquelle $R_{11}$ et $R_{12}$ ont la même définition que dans la formule (I), pour conduire aux composés de formule (XII) :

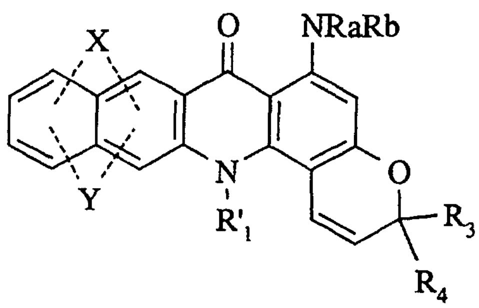

**(XII)**

dans laquelle X, Y, R'$_1$, $R_3$, $R_4$, Ra et Rb sont tels que définis précédemment,
l'ensemble des composés de formule (VI), (IX), (X) et (XII) formant les composés de formule (XIII) :

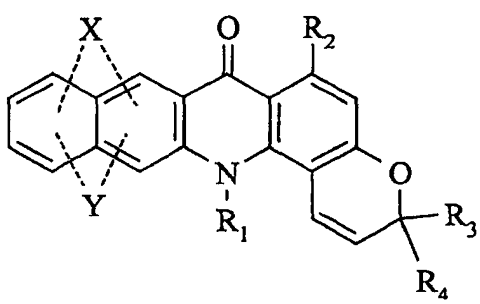

**(XIII)**

dans laquelle X, Y, $R_1$, $R_2$, $R_3$ et $R_4$ ont la même signification que dans la définition générale de la formule (I), composés de formule (XIII) qui sont soumis,

a) ➪ soit à l'action du tétroxyde d'osmium en milieu polaire et en présence de 4-méthylmorpholine-N-oxyde, pour conduire aux composés de formules (XIV/a) et (XIV/b) :

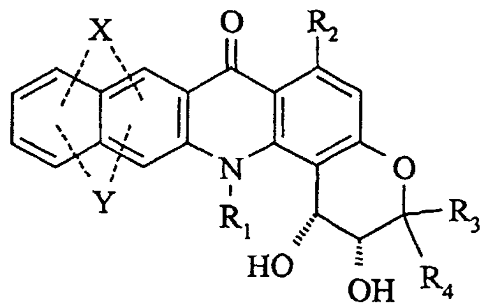

(XIV/a)

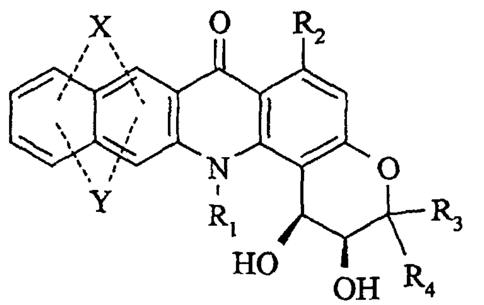

(XIV/b)

dans lesquelles X, Y, $R_1$, $R_2$, $R_3$ et $R_4$ sont tels que définis précédemment,
les composés de formule (XIV/a) et (XIV/b) pouvant aussi être obtenus séparément par synthèse chirale et notamment par *cis* dihydroxylation asymétrique à partir du composé (XIII) en utilisant des ligands chiraux,
b) ⇒ soit à l'action du permanganate de potassium en milieu polaire, pour conduire aux composés de formule (XV) :

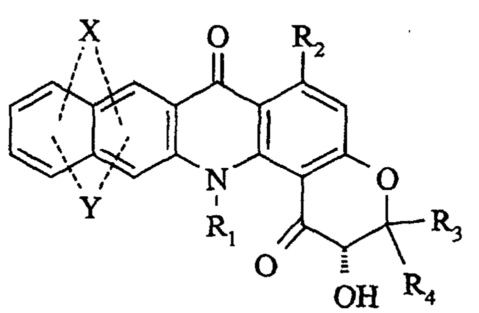

(XV)

dans laquelle X, Y, $R_1$, $R_2$, $R_3$ et $R_4$ sont tels que définis précédemment,
composés de formule (XV) qui sont soumis à des conditions réductrices en présence de $NaBH_4$ par exemple, pour conduire aux composés de formule (XIV/c) :

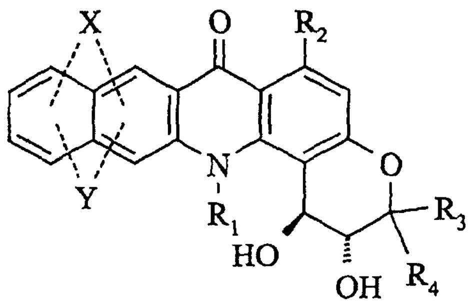

**(XIV/c)**

dans laquelle X, Y, $R_1$, $R_2$, $R_3$ et $R_4$ sont tels que définis précédemment,
l'ensemble des composés de formules (XIV/a), (XIV/b) et (XIV/c) formant les composés de formule (XIV) :

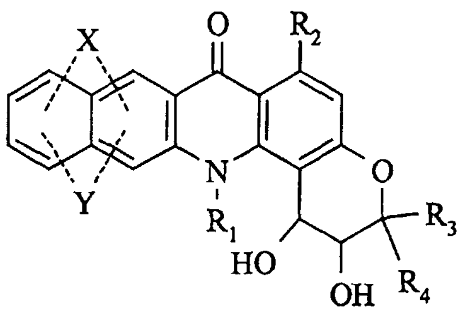

**(XIV)**

dans laquelle X, Y, $R_1$, $R_2$, $R_3$ et $R_4$ sont tels que définis précédemment,
composés de formule (XIV) qui sont soumis :

- soit à l'action d'un alcool de formule $R_{20}$-OH dans laquelle $R_{20}$ représente un groupement alkyle ($C_1$-$C_6$) linéaire ou ramifié,
pour conduire aux composés de formule (XVI/a) :

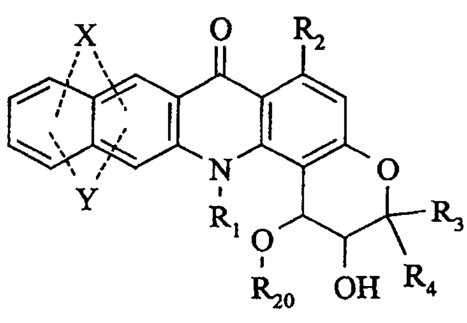

**(XVI/a)**

dans laquelle X, Y, $R_1$, $R_2$, $R_3$, $R_4$ et $R_{20}$ sont tels que définis précédemment,
composés de formule (XVI/a) dont la fonction alcool est estérifiée en présence d'un composé de formule

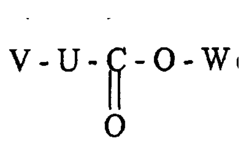

ou $(V\text{-}U\text{-}CO)_2O$ dans lesquelles U et V sont tels que définis dans la formule (I) et W représente un

groupement partant,
pour conduire aux composés de formule (I/a), cas particulier des composés de formule (I) :

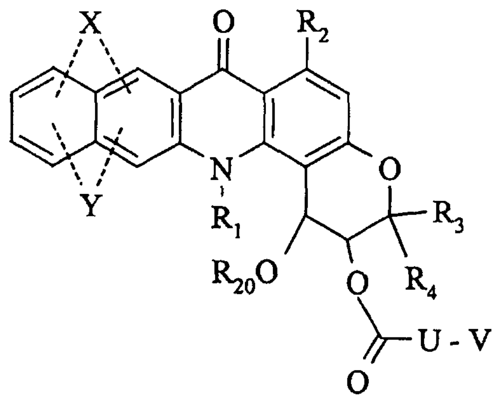

**(I/a)**

dans laquelle X, Y, $R_1$, $R_2$, $R_3$, $R_4$, $R_{20}$, U et V sont tels que définis précédemment,

❖ soit à l'action d'un iodure d'alkyle de formule $R'_{20}$-I dans laquelle $R'_{20}$ représente un groupement alkyle ($C_1$-$C_6$) linéaire ou ramifié, en présence de sel d'Argent, pour conduire aux composés de formule (XVI/b) :

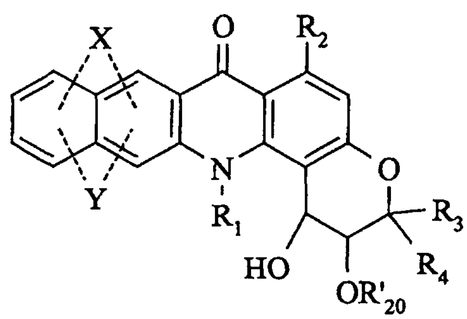

**(XVI/b)**

cas particulier des composés de formule (I), dans laquelle X, Y, $R_1$, $R_2$, $R_3$, $R_4$ et $R'_{20}$ sont tels que définis précédemment,
composés de formule (XVI/b) dont la fonction alcool est estérifiée en présence d'un composé de formule

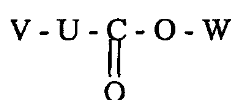

ou $(V\text{-}U\text{-}CO)_2O$ tels que définis précédemment, pour conduire aux composés de formule (I/b) :

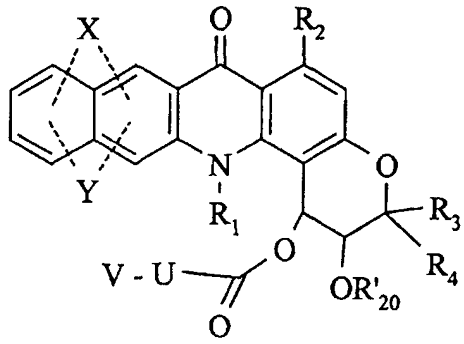

(I/b)

cas particulier des composés de formule (I), dans laquelle X, Y, $R_1$, $R_2$, $R_3$, $R_4$, $R'_{20}$, U et V sont tels que définis précédemment,

- soit à l'action directe d'un composé de formule

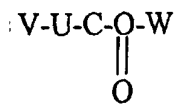

ou $(V\text{-}U\text{-}CO)_2O$,
tels que définis précédemment, en présence d'une base, afin d'obtenir les composés de formule (I/c), cas particulier des composés de formule (I) :

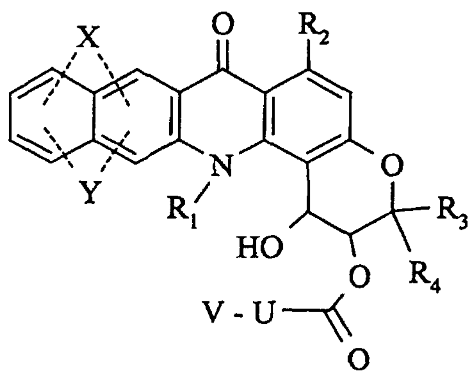

(I/c)

dans laquelle X, Y, $R_1$, $R_2$, $R_3$, $R_4$, U et V sont tels que définis précédemment, composé de formule (I/c), qui peut être soumis à nouveau, dans les mêmes conditions opératoires, à l'action d'un anhydride de formule $(R_{30}CO)_2O$ dans laquelle $R_{30}$ représente un groupement alkyle ($C_1$-$C_6$) linéaire ou ramifié, aryle ou arylalkyle ($C_1$-$C_6$) linéaire ou ramifié, pour conduire aux composés de formule (I/d) :

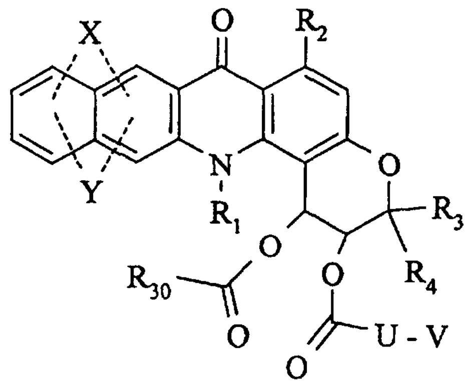

**(I/d)**

dans laquelle X, Y, $R_1$, $R_2$, $R_3$, $R_4$, $R_{30}$, U et V sont tels que définis précédemment, ou composé de formule (I/c) qui peut être traité de nouveau avec un composé de formule

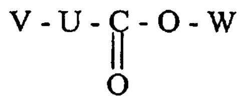

ou $(V\text{-}U\text{-}CO)_2O$ tels que définis précédemment, pour conduire aux composés de formule (I/e), cas particulier des composés de formule (I) :

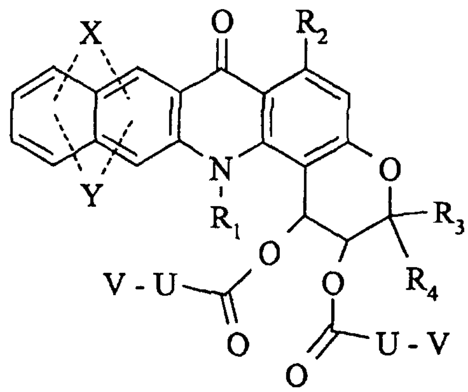

**(I/e)**

dans laquelle X, Y, $R_1$, $R_2$, $R_3$, $R_4$, U et V ont la même signification que dans la formule (I), étant entendu que les deux groupements U et les deux groupements V peuvent être chacun identique ou différent,

c) ⇒ soit à l'action d'un peracide, pour conduire au composé de formule (XVII) :

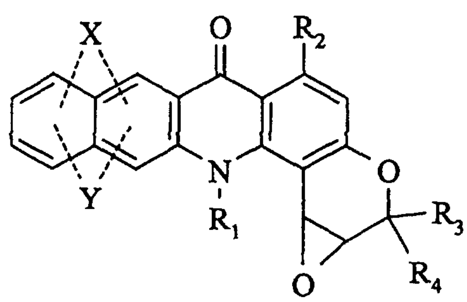

**(XVII)**

dans laquelle X, Y, $R_1$, $R_2$, $R_3$ et $R_4$, sont tels que définis précédemment, composés de formule (XVII) qui est traité éventuellement par de l'ammoniac ou une amine primaire ou

secondaire pour conduire aux composés de formule (XVIII/a) et/ou (XVIII/b) suivant la nature des réactifs :

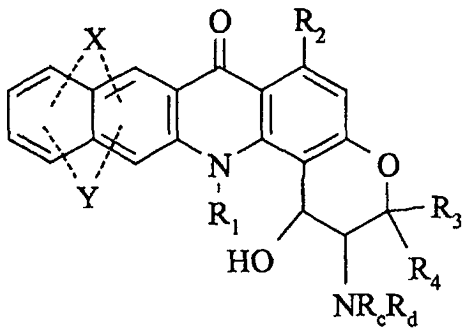

(XVIII/a)

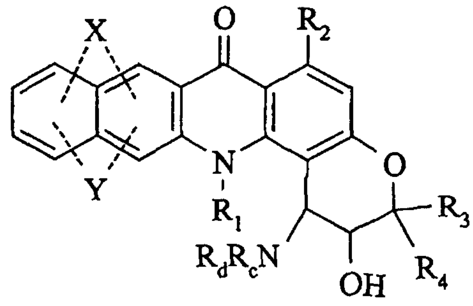

(XVIII/b)

dans lesquelles X, Y, $R_1$, $R_2$, $R_3$ et $R_4$, sont tels que définis précédemment, et $R_c$ et $R_d$ représentent un atome d'hydrogène ou un groupement alkyle ($C_1$-$C_6$) linéaire ou ramifié, composés de formules (XVIII/a) et (XVIII/b) qui sont mis en présence d'un composé de formule

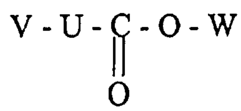

tel que défini précédemment, pour conduire respectivement aux composés de formules (I/f) et (I/g), cas particuliers des composés de formule (I) :

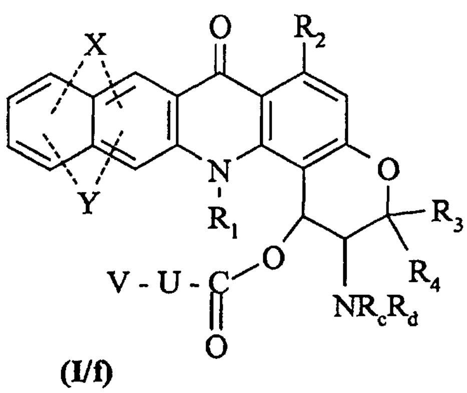

(I/f)

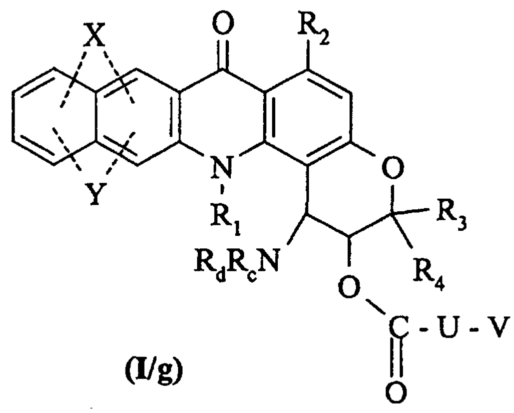

(I/g)

dans lesquelles X, Y, $R_1$, $R_2$, $R_3$, $R_4$, $R_c$, $R_d$, U et V sont tels que définis précédemment,
les composés (I/a) à (I/g) formant l'ensemble des composés de l'invention qui sont purifiés, le cas échéant, selon une technique classique de purification, qui peuvent, si on le désire, être séparés en leurs différents isomères optiques selon the technique classique de séparation et qui sont transformés, si on le souhaite, en leurs N-oxydes et, le cas échéant, en leurs sels d'addition à un acide ou à une base pharmaceutiquement acceptable.

**11.** Compositions pharmaceutiques contenant comme principe actif au moins un composé selon l'une quelconque des revendications 1 à 9, seul ou en combinaison avec un ou plusieurs excipients ou véhicules inertes non toxiques pharmaceutiquement acceptables.

**12.** Compositions pharmaceutiques selon la revendication 11 contenant au moins un principe actif selon l'une quelconque des revendications 1 à 9 utiles dans le traitement des cancers.

## Claims

**1.** Compounds of formula (I) :

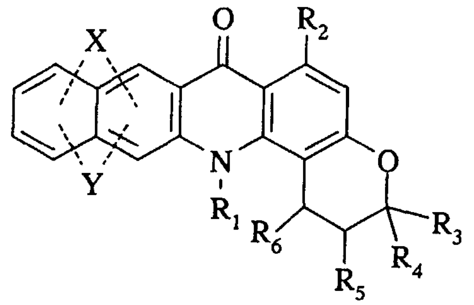

(I)

wherein:

X and Y, which may be identical or different, each independently of the other represents a group selected from a hydrogen atom, a halogen atom, a hydroxy group, a mercapto group, a cyano group, a nitro group, a linear or branched $(C_1-C_6)$alkyl group, a linear or branched $(C_1-C_6)$alkoxy group, a trihalo-$(C_1-C_6)$alkyl group in which the alkyl moiety is linear or branched, and an amino group optionally substituted by one or two identical or different linear or branched $(C_1-C_6)$alkyl groups which may themselves be substituted by a linear or branched $(C_1-C_6)$alkoxy group or by a group of formula $-NR_7R_8$ wherein $R_7$ and $R_8$, which may be identical or different, each independently of the other represents a hydrogen atom, a linear or branched $(C_1-C_6)$alkyl group, an aryl group or an aryl-$(C_1-C_6)$alkyl group in which the alkyl moiety is linear or branched, or X and Y together form a methylenedioxy or ethylenedioxy group, it being understood that the substituents X and Y may be present on either of the two adjacent benzene rings,

$R_1$ represents a hydrogen atom or a linear or branched $(C_1-C_6)$alkyl group,

$R_2$ represents :

- a hydrogen atom,
- a hydroxy group,
- a linear or branched $(C_1-C_6)$alkyl group,
- a linear or branched $(C_1-C_6)$alkoxy group optionally substituted by a group selected from :
    * a group of formula $NR_9R_{10}$ wherein $R_9$ and $R_{10}$, which may be identical or different, each independently of the other represents a hydrogen atom, a linear or branched $(C_1-C_6)$alkyl group, or a linear or branched $(C_1-C_6)$hydroxyalkyl group, and
    * a saturated or unsaturated monocyclic or bicyclic heterocycle having from 5 to 7 ring members containing one or two hetero atoms selected from oxygen, nitrogen and sulphur,
- a linear or branched $(C_1-C_6)$alkylcarbonyloxy group, or
- an amino group optionally substituted by :
    * one or two identical or different groups, linear or branched $(C_1-C_6)$alkyl, aryl or aryl-$(C_1-C_6)$alkyl in which the alkyl moiety is linear or branched,
    * a linear or branched $(C_1-C_6)$alkylcarbonyl group optionally substituted by a group $-NR_7R_8$ wherein $R_7$ and $R_8$ are as defined hereinbefore,
    * a group of formula $-R_{11}-NR_9R_{10}$, wherein $R_{11}$ represents a linear or branched $(C_1-C_6)$ alkylene group, and $R_9$ and $R_{10}$, which may be identical or different, each independently of the other represents a hydrogen atom, a linear or branched $(C_1-C_6)$alkyl group, or a linear or branched $(C_1-C_6)$hydroxyalkyl group,
    * a linear or branched $(C_1-C_6)$alkylene group, substituted by a saturated or unsaturated monocyclic or bicyclic heterocycle having from 5 to 7 ring members containing one or

two hetero atoms selected from oxygen, nitrogen and sulphur, or

* by a group of formula $-R_{11}-CO-R_{12}$ wherein $R_{11}$ is as defined hereinbefore, and $R_{12}$ represents a hydroxy group or a linear or branched $(C_1-C_6)$alkoxy group,

$R_3$ and $R_4$, which may be identical or different, each independently of the other represents a hydrogen atom or a linear or branched $(C_1-C_6)$alkyl group,

$R_5$ and/or $R_6$ represent(s) a group of formula -O-CO-U-V wherein :

- U represents a linear or branched $(C_1-C_8)$alkylene chain, optionally substituted by one or more identical or different groups selected from aryl, hydroxy and linear or branched $(C_1-C_6)$ alkoxy,
- V represents a group selected from :
  * carboxy,
  * $-CO_2R_{13}$ wherein $R_{13}$ represents a linear or branched $(C_1-C_6)$alkyl group optionally substituted by one or more hydroxy groups, an aryl group or an aryl-$(C_1-C_6)$alkyl group in which the alkyl moiety is linear or branched,
  * hydroxy,
  * linear or branched $(C_1-C_6)$alkoxy,
  * $-NR_7R_8$ wherein $R_7$ and $R_8$, which may be identical or different, are as defined hereinbefore,
  * $-NR_7-CO_2R_{13}$ wherein $R_7$ and $R_{13}$ are as defined hereinbefore,
  * $-NR_7-COR_{13}$ wherein $R_7$ and $R_{13}$ are as defined hereinbefore,
  * $-COR_{13}$ wherein $R_{13}$ is as defined hereinbefore, and
  * $-CO-NR_7R_8$ wherein $R_7$ and $R_8$, which may be identical or different, are as defined hereinbefore,

and when only one of the two groups $R_5$ and $R_6$ represents a group of formula -O-CO-U-V, the other of the $R_5$ and $R_6$ groups represents a group Z selected from :

- hydroxy,
- linear or branched $(C_1-C_6)$alkoxy,
- linear or branched $(C_1-C_6)$alkylcarbonyloxy,
- arylcarbonyloxy,
- aryl-$(C_1-C_6)$alkylcarbonyloxy in which the alkyl moiety is linear or branched, and
- amino optionally substituted by one or two identical or different linear or branched $(C_1-C_6)$alkyl groups,

their optical isomers, N-oxides, and addition salts thereof with a pharmaceutically acceptable acid or base, it being understood that "aryl" is understood to mean a phenyl or naphthyl group, optionally containing one or more identical or different substituents selected from hydroxy, halo, carboxy, nitro, amino, $(C_1-C_6)$alkylamino or di-$(C_1-C_6)$alkylamino in which the(each) alkyl moiety is linear or branched, linear or branched $(C_1-C_6)$alkoxy, linear or branched $(C_1-C_6)$acyl, and linear or branched $(C_1-C_6)$alkylcarbonyloxy.

2. Compounds of formula (I) according to claim 1 **characterised in that** $R_3$ and $R_4$, which may be identical or different, represent a linear or branched $(C_1-C_6)$alkyl group, their optical isomers, N-oxides, and addition salts thereof with a pharmaceutically acceptable acid or base.

3. Compounds of formula (I) according to any one of claims 1 or 2, **characterised in that** $R_3$ and $R_4$, which are identical, each represents a methyl group, their optical isomers, N-oxides, and addition salts thereof with a pharmaceutically acceptable acid or base.

4. Compounds of formula (I) according to claim 1, **characterised in that** $R_2$ represents a linear or branched $(C_1-C_6)$ alkoxy group, or an optionally substituted amino group as defined for formula (I), their optical isomers, N-oxides, and addition salts thereof with a pharmaceutically acceptable acid or base.

5. Compounds of formula (I) according to claim 1, **characterised in that** $R_5$ represents a group of formula -O-CO-U-V wherein U and V are as defined for formula (I) and $R_6$ represents a Z group as defined for formula (I), their optical

isomers, N-oxides, and addition salts thereof with a pharmaceutically acceptable acid or base.

**6.** Compounds of formula (I) according to claim 1, **characterised in that** $R_6$ and $R_5$, which are identical, each represents a group of formula -O-CO-U-V wherein U and V are as defined for formula (I), their optical isomers, N-oxides, and addition salts thereof with a pharmaceutically acceptable acid or base.

**7.** Compounds of formula (I) according to any one of claims 1 or 5, **characterised in that** $R_5$ represents a group of formula -O-CO-U-V wherein U represents a linear ($C_1$-$C_4$)alkylene chain and V represents a group selected from carboxy, -$NR_7R_8$, -$NR_7$-$CO_2R_{13}$ and

- $NR_7$-$COR_{13}$ wherein $R_7$, $R_8$ and $R_{13}$, which may be identical or different, represent a hydrogen atom or a linear or branched ($C_1$-$C_6$)alkyl group, their optical isomers, N-oxides, and addition salts thereof with a pharmaceutically acceptable acid or base.

**8.** Compounds of formula (I) according to claim 1, **characterised in that** $R_6$ represents a linear or branched ($C_1$-$C_6$) alkylcarbonyloxy group, their optical isomers, N-oxides, and addition salts thereof with a pharmaceutically acceptable acid or base.

**9.** Compounds of formula (I) according to claim 1 which are:

- (±)-*cis*-4-{[1-(acetyloxy)-6-methoxy-3,3,14-trimethyl-7-oxo-2,3,7,14-tetrahydro-1*H*-benzo[*b*]pyrano[3,2-h] acridin-2-yl]oxy}-4-oxobutanoic acid,
- (±)-*cis*-4-({1-[(3-carboxypropanoyl)oxy]-6-methoxy-3,3,14-trimethyl-7-oxo-2,3,7,14-tetrahydro-1*H*-benzo[*b*] pyrano[3,2-*h*]acridin-2-yl}oxy)-4-oxobutanoic acid,
- (±)-*cis*-5-{[1-(acetyloxy)-6-methoxy-3,3,14-trimethyl-7-oxo-2,3,7,14-tetrahydro-1*H*-benzo[b]pyrano[3,2-*h*] acridin-2-yl]oxy}-5-oxopentanoic acid,
- *cis*-[1-(acetyloxy)-6-methoxy-3,3,14-trimethyl-7-oxo-2,3,7,14-tetrahydro-1*H*-benzo[*b*]-pyrano[3,2-*h*]acridin-2-yl] (dimethylamino)acetate, and
- *cis*-[1-(acetyloxy)-6-methoxy-3,3,14-trimethyl-7-oxo-2,3,7,14-tetrahydro-1*H*-benzo[*b*]-pyrano[3,2-*h*]acridin-2-yl ] 4-(dimethylamino)butanoate,

their optical isomers, N-oxides, and addition salts thereof with a pharmaceutically acceptable acid or base.

**10.** Process for the preparation of compounds of formula (I) according to claim 1, **characterised in that** :

- *either a 3-amino-2-naphthalenecarboxylic acid compound (II) :*

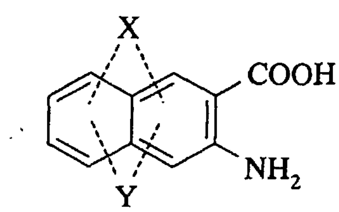

**(II)**

wherein X and Y are as defined for formula (I) ,
is reacted with a phloroglucinol compound of formula (III) :

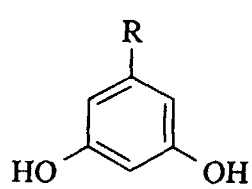

**(III)**

wherein R represents a hydrogen atom, a hydroxy group or a linear or branched ($C_1$-$C_6$)-alkyl group,
to yield the compounds of formula (IV) :

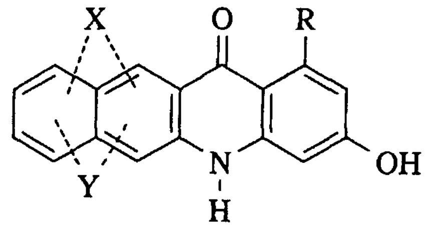

(IV)

wherein X, Y and R are as defined hereinbefore, which are then treated under basic conditions in an aprotic solvent with an alkyne of formula (V) :

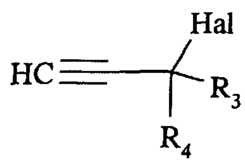

(V)

wherein Hal represents a halogen atom and $R_3$ and $R_4$ are as defined for formula (I), to yield the compounds of formula (VI) :

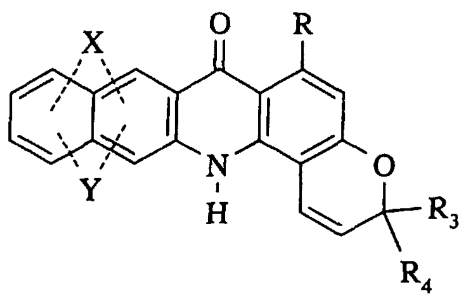

(VI)

wherein X, Y, R, $R_3$ and $R_4$ are as defined hereinbefore,

- *or a 3-halo-2-naphthalenecarboxylic acid compound of formula (VII) :*

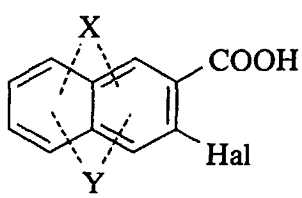

(VII)

wherein X and Y are as defined for formula (I) and Hal represents a halogen atom, is reacted with an amino-chromene compound of formula (VIII) :

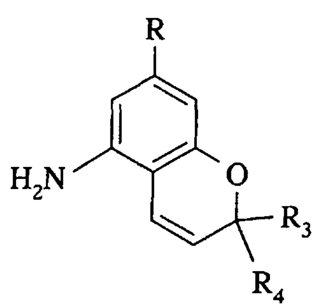

(VIII)

wherein $R_3$ and $R_4$ are as defined for formula (I) and R is as defined hereinbefore, also to yield the compounds of formula (VI) :

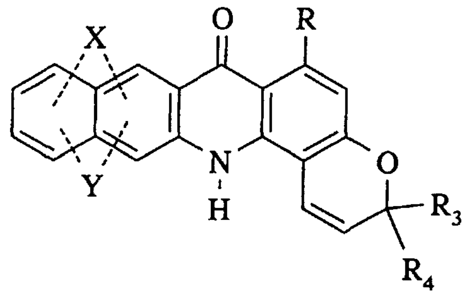

**(VI)**

wherein X, Y, R, $R_3$ and $R_4$ are as defined hereinbefore,

the nitrogen atom of which compounds of formula (VI) is optionally substituted, by the action of an alkyl halide or a dialkyl sulphate in the presence of a deprotonating agent, in an aprotic polar solvent, to yield the compounds of formula (IX):

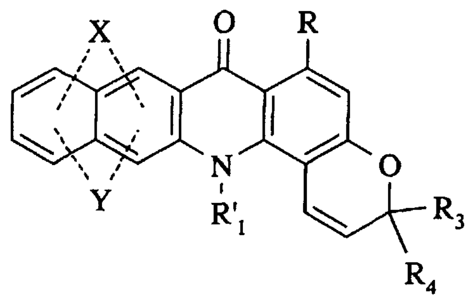

**(IX)**

wherein X, Y, R, $R_3$ and $R_4$ are as defined hereinbefore, and $R'_1$ represents a linear or branched ($C_1$-$C_6$)alkyl group, which compounds of formula (IX) are subjected to the action of an alkylating agent, such as a dialkyl sulphate, or of an acylating agent, to yield the compounds of formula (X) :

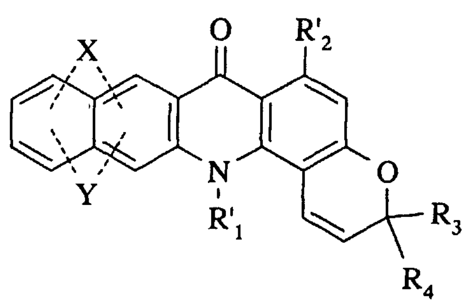

**(X)**

wherein X, Y, $R'_1$, $R_3$ and $R_4$ are as defined hereinbefore and $R'_2$ represents an alkoxy group (optionally substituted by a group of formula $NR_9R_{10}$ wherein $R_9$ and $R_{10}$ are as defined for formula (I)), or a linear or branched ($C_1$-$C_6$) alkylcarbonyloxy group,
which compound of formula (X), when $R'_2$ represents an alkoxy group for example, is treated with a compound of formula (XI) :

HNRaRb **(XI)**

wherein Ra represents a hydrogen atom, a linear or branched ($C_1$-$C_6$)alkyl group, an aryl group, or an aryl-($C_1$-$C_6$) alkyl group in which the alkyl moiety is linear or branched, and Rb represents a hydrogen atom, a linear or branched

$(C_1-C_6)$alkyl group, an aryl group, an aryl-$(C_1-C_6)$alkyl group in which the alkyl moiety is linear or branched, a group of formula $-R_{11}-NR_9R_{10}$ wherein $R_{11}$, $R_{10}$ and $R_9$ are as defined for formula (I), a linear or branched $(C_1-C_6)$alkyl-carbonyl group in which the alkyl moiety is optionally substituted by a group $NR_7R_8$ as defined for formula (I), a heterocycloalkylene group (the terms "alkylene" and "heterocycle" being as defined for formula (I)) or a group of formula $-R_{11}-CO-R_{12}$ wherein $R_{11}$ and $R_{12}$ are as defined for formula (I),
to yield the compounds of formula (XII) :

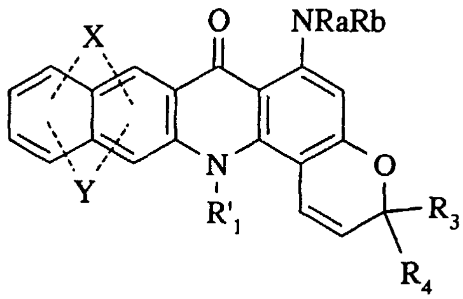

(XII)

wherein X, Y, $R'_1$, $R_3$, $R_4$, Ra and Rb are as defined hereinbefore,
the totality of the compounds of formulae (VI), (IX), (X) and (XII) constituting the compounds of formula (XIII) :

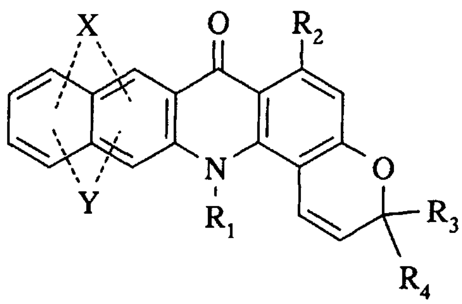

(XIII)

wherein X, Y, $R_1$, $R_2$, $R_3$ and $R_4$ are as defined in the general definition of formula (I),
which compounds of formula (XIII) are subjected

a) ⇨ either to the action of osmium tetroxide in a polar medium and in the presence of 4-methylmorpholine-N-oxide, to yield the compounds of formulae (XIV/a) and (XIV/b) :

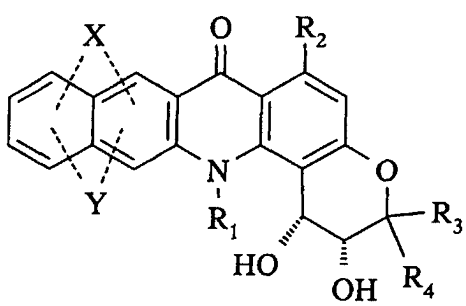

(XIV/a)

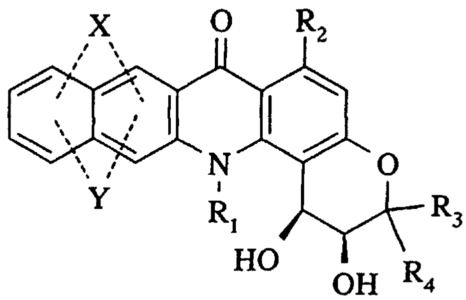

(XIV/b)

wherein X, Y, $R_1$, $R_2$, $R_3$ and $R_4$ are as defined hereinbefore,

it also being possible for the compounds of formulae (XIV/a) and (XIV/b) to be obtained separately by chiral synthesis and especially by asymmetric *cis* dihydroxylation starting from compound (XIII) using chiral ligands,

b) ⇨ or to the action of potassium permanganate in a polar medium, to yield the compounds of formula (XV) :

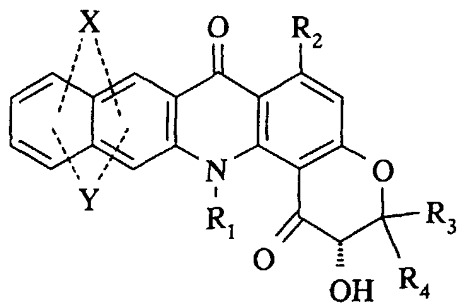

(XV)

wherein X, Y, $R_1$, $R_2$, $R_3$ and $R_4$ are as defined hereinbefore,

which compounds of formula (XV) are subjected to reductive conditions in the presence of $NaBH_4$ for example, to yield the compounds of formula (XIV/c) :

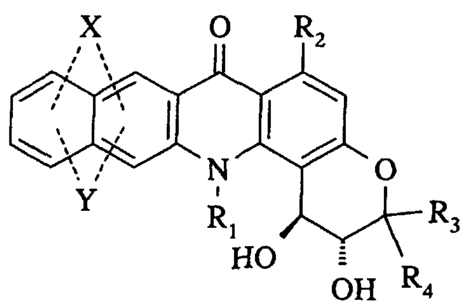

(XIV/c)

wherein X, Y, $R_1$, $R_2$, $R_3$ and $R_4$ are as defined hereinbefore,

the totality of the compounds of formulae (XIV/a), (XIV/b) and (XIV/c) constituting the compounds of formula (XIV) :

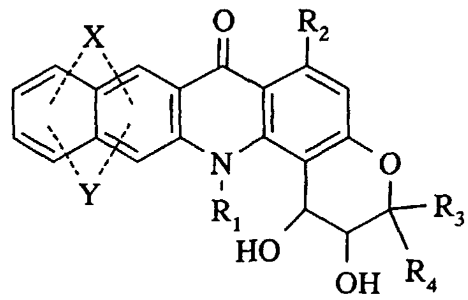

(XIV)

wherein X, Y, $R_1$, $R_2$, $R_3$ and $R_4$ are as defined hereinbefore, which compounds of formula (XIV) are subjected :

- ❖ either to the action of an alcohol of formula $R_{20}$-OH wherein $R_{20}$ represents a linear or branched ($C_1$-$C_6$) alkyl group,

  to yield the compounds of formula (XVI/a) :

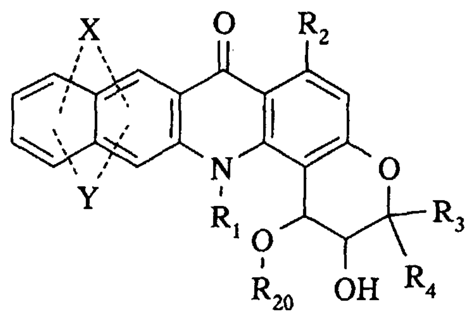

(XVI/a)

wherein X, Y, $R_1$, $R_2$, $R_3$, $R_4$ and $R_{20}$ are as defined hereinbefore, the alcohol function of which compounds of formula (XVI/a) is esterified in the presence of a compound of formula

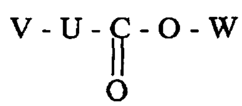

or $(V\text{-}U\text{-}CO)_2O$ wherein U and V are as defined for formula (I) and W represents a leaving group, to yield the compounds of formula (I/a), a particular case of the compounds of formula (I) :

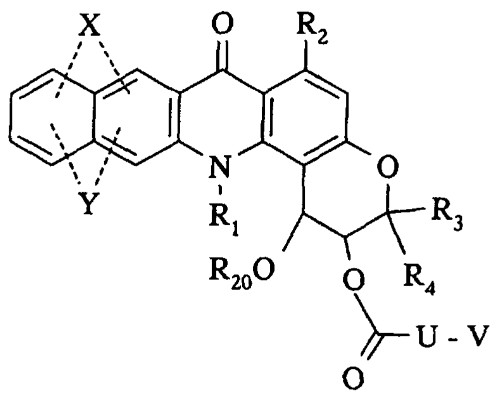

(I/a)

wherein X, Y, $R_1$, $R_2$, $R_3$, $R_4$, $R_{20}$, U and V are as defined hereinbefore,

❖ or to the action of an alkyl iodide of formula $R'_{20}$-I wherein $R'_{20}$ represents a linear or branched ($C_1$-$C_6$) alkyl group, in the presence of a silver salt, to yield the compounds of formula (XVI/b) :

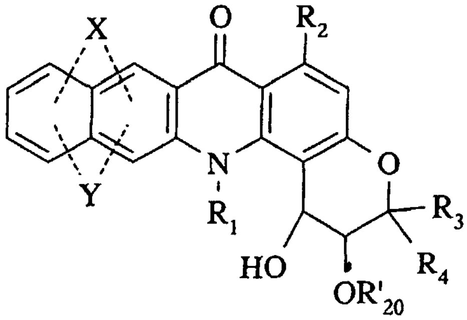

**(XVI/b)**

a particular case of the compounds of formula (I), wherein X, Y, $R_1$, $R_2$, $R_3$, $R_4$ and $R'_{20}$ are as defined hereinbefore,

the alcohol function of which compounds of formula (XVI/b) is esterified in the presence of a compound of formula

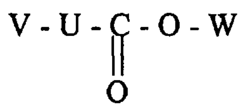

or $(V\text{-}U\text{-}CO)_2O$ as defined hereinbefore, to yield the compounds of formula (I/b) :

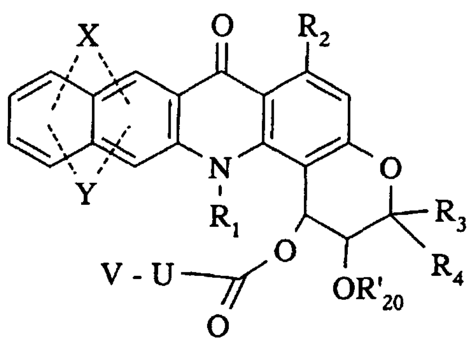

**(I/b)**

a particular case of the compounds of formula (I), wherein X, Y, $R_1$, $R_2$, $R_3$, $R_4$, $R'_{20}$, U and V are as defined hereinbefore,

❖ or to the direct action of a compound of formula

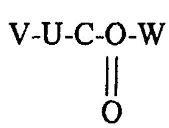

or $(V\text{-}U\text{-}CO)_2O$, as defined hereinbefore, in the presence of a base, in order to obtain the compounds of formula (I/c), a particular case of the compounds of formula (I):

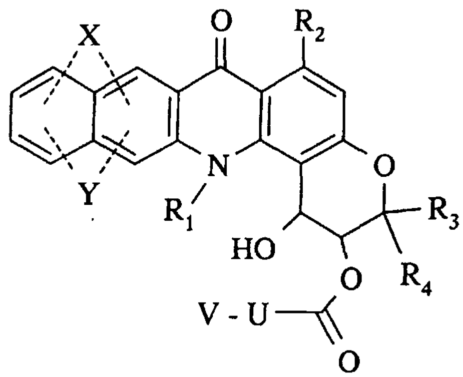

(I/c)

wherein X, Y, $R_1$, $R_2$, $R_3$, $R_4$, U and V are as defined hereinbefore, which compound of formula (I/c) may be subjected again, under the same operating conditions, to the action of an anhydride of formula $(R_{30}CO)_2O$ wherein $R_{30}$ represents a linear or branched $(C_1\text{-}C_6)$alkyl group, an aryl group or an aryl-$(C_1\text{-}C_6)$-alkyl group in which the alkyl moiety is linear or branched, to yield the compounds of formula (I/d) :

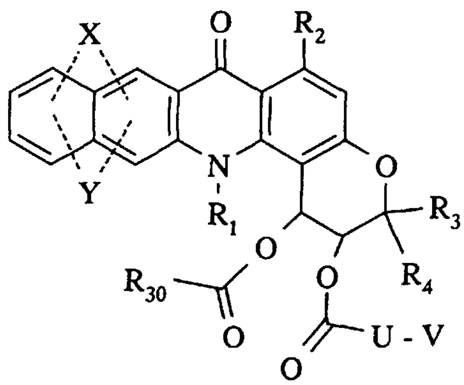

(I/d)

wherein X, Y, $R_1$, $R_2$, $R_3$, $R_4$, $R_{30}$, U and V are as defined hereinbefore, or which compound of formula (I/c) may be treated again with a compound of formula

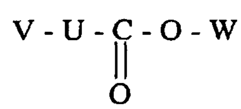

or $(V\text{-}U\text{-}CO)_2O$ as defined hereinbefore, to yield the compounds of formula (I/e), a particular case of the compounds of formula (I) :

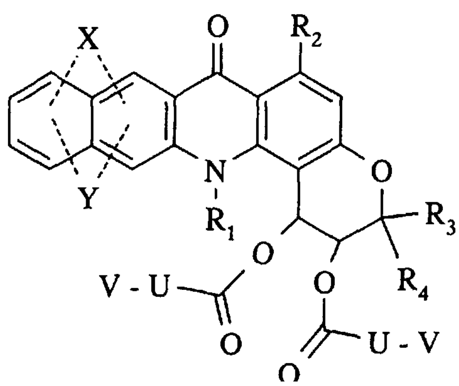

(I/e)

wherein X, Y, $R_1$, $R_2$, $R_3$, $R_4$, U and V are as defined for formula (I), it being understood that the two U groups and the two V groups may each be identical or different,

c) ➪ or to the action of a peracid, to yield the compound of formula (XVII) :

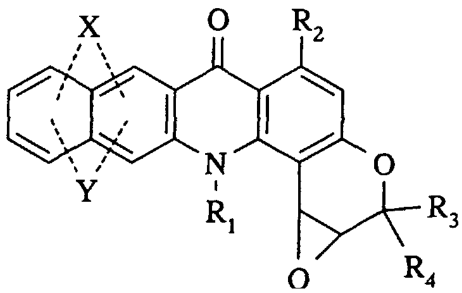

**(XVII)**

wherein X, Y, $R_1$, $R_2$, $R_3$ and $R_4$ are as defined hereinbefore,
which compound of formula (XVII) is optionally treated with ammonium hydroxide or a primary or secondary amine to yield the compounds of formula (XVIII/a) and/or (XVIII/b) depending upon the nature of the reagents :

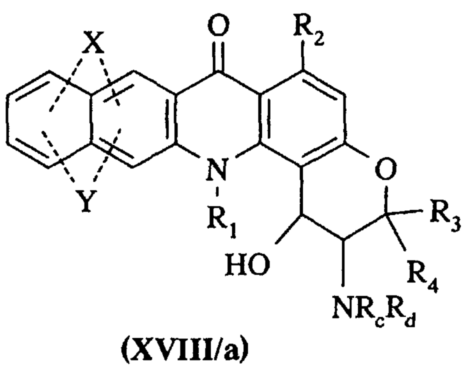

**(XVIII/a)**

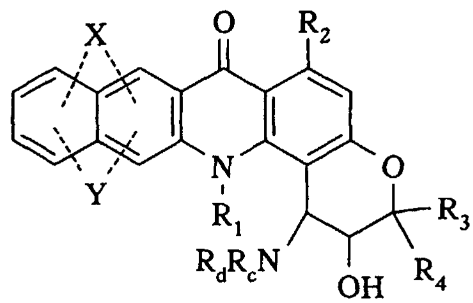

**(XVIII/b)**

wherein X, Y, $R_1$, $R_2$, $R_3$ and $R_4$ are as defined hereinbefore, and $R_c$ and $R_d$ represent a hydrogen atom or a linear or branched $(C_1\text{-}C_6)$alkyl group,
which compounds of formulae (XVIII/a) and (XVIII/b) are treated with a compound of formula

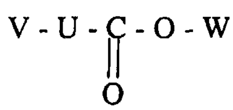

as defined hereinbefore, to yield the compounds of formulae (I/f) and (I/g), respectively, particular cases of the compounds of formula (I) :

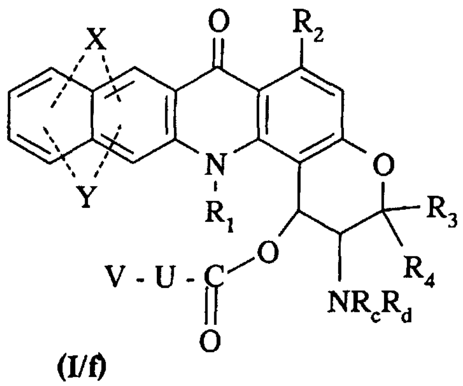

(I/f)

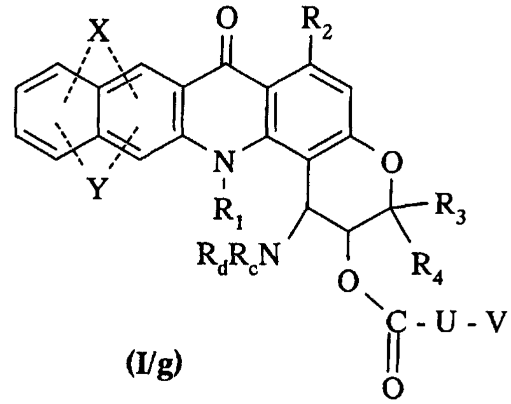

(I/g)

wherein X, Y, $R_1$, $R_2$, $R_3$, $R_4$, $R_c$, $R_d$, U and V are as defined hereinbefore,
which compounds (I/a) to (I/g) constitute the totality of the compounds of the invention, which are purified, if necessary, according to a conventional purification technique, may be separated, if desired, into their various optical isomers according to a conventional separation technique, and which are converted, if desired, into N-oxides thereof and, where appropriate, into addition salts thereof with a pharmaceutically acceptable acid or base.

**11.** Pharmaceutical compositions comprising as active ingredient at least one compound according to any one of claims 1 to 9, alone or in combination with one or more inert, nontoxic, pharmaceutically acceptable excipients or carriers.

**12.** Pharmaceutical compositions according to claim 11 comprising at least one active ingredient according to any one of claims 1 to 9 for use in the treatment of cancer.

## Patentansprüche

**1.** Verbindungen der Formel (I):

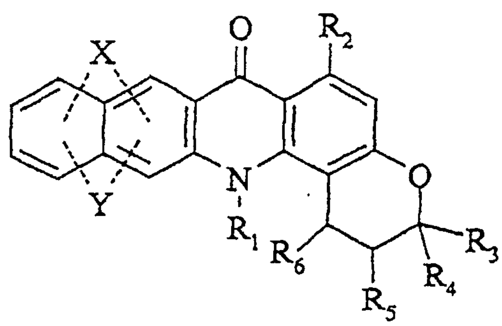

(I)

in der:

X, Y, die gleichartig oder verschieden sind, unabhängig voneinander eine Gruppe ausgewählt aus Wasserstoffatomen, Halogenatomen, Hydroxygruppen, Mercaptogruppen, Cyanogruppen, Nitrogruppen, geradkettigen oder verzweigten ($C_1$-$C_6$)-Alkylgruppen, geradkettigen oder verzweigten ($C_1$-$C_6$)-Alkoxygruppen, geradkettigen oder verzweigten ($C_1$-$C_6$)-Trihalogenalkylgruppen, Aminogruppen. die gegebenenfalls durch eine oder zwei gleichartige oder verschiedene, gerakdetige oder verzweigte ($C_1$-$C_6$)-Alkylgruppen substituiert sind, die ihrerseits gegebenenfalls durch eine geradkettige oder verzweigte ($C_1$-$C_6$)-Alkoxygruppe oder eine Gruppe der Formel $-NR_7R_8$, in der $R_7$ und $R_8$, die gleichartig oder verschieden sind, unabhängig voneinander Wasserstoffatome, geradkettige oder verzweigte ($C_1$-$C_6$)-Alkylgruppen, Arylgruppen oder ge-

radkettige oder verzweigte Aryl-($C_1$-$C_6$)-alkylgruppen darstellen, substituiert sind, bedeuten oder gemeinsam eine Methylendioxygruppe oder eine Ethylendioxygruppe bilden, mit der Maßgabe, daß die Substituenten X und Y an dem einen oder dem anderen der beiden benachbarten Benzolringe vorhanden sein können,

$R_1$ ein Wasserstoffatom oder eine geradkettige oder verzweigte ($C_1$-$C_6$)-Alkylgruppe darstellt,

$R_2$: - ein Wasserstoffatom,

- eine Hydroxygruppe,
- eine geradkettige oder verzweigte ($C_1$-$C_6$)-Alkylgruppe,
- eine geradkettige oder verzweigte ($C_1$-$C_6$)-Alkoxygruppe, die gegebenenfalls durch eine Gruppe substituiert ist ausgewählt aus:

  * Gruppen der Formel $NR_9R_{10}$, worin $R_9$ und $R_{10}$, die gleichartig oder verschieden sind, unabhängig voneinander Wasserstoffatome, geradkettige oder verzweigte ($C_1$-$C_6$)-Alkylgruppen oder geradkettige oder verzweigte ($C_1$-$C_6$)-Hydroxyalkylgruppen darstellen,
  * und gesättigten oder ungesättigten, monocyclischen oder bicyclischen Heterocyclen mit 5 bis 7 Kettengliedern, die ein oder zwei Heteroatome ausgewählt aus Sauerstoff, Stickstoff und Schwefel enthalten,

- eine geradkettige oder verzweigte ($C_1$-$C_6$)-Alkylcarbonyloxygruppe,
- oder eine Aminogruppe gegebenenfalls substituiert:

  * durch eine oder zwei gleichartige oder verschiedene, geradkettige oder verzweigte ($C_1$-$C_6$)-Alkylgruppen, Arylgruppen oder geradkettige oder verzweigte Aryl-($C_1$-$C_6$)-alkylruppen,
  * durch eine geradkettige oder verzweigte ($C_1$-$C_6$)-Alkylcarbonylgruppe, die gegebenenfalls durch eine Gruppe $-NR_7R_8$ substituiert ist, worin $R_7$ und $R_8$ die oben angegebenen Bedeutungen besitzen,
  * durch eine Gruppe der Formel $-R_{11}-NR_9R_{10}$, worin $R_{11}$ eine geradkettige oder verzweigte ($C_1$-$C_6$)-Alkylengruppe und $R_9$ und $R_{10}$, die gleichartig oder verschieden sind, unabhängig voneinander Wasserstoffatome, geradkettige oder verzweigte ($C_1$-$C_6$)-Alkylgruppen oder geradkettige oder verzweigte ($C_1$-$C_6$)-Hydroxyalkylgruppen darstellen,
  * durch eine geradkettige oder verzweigte ($C_1$-$C_6$)-Alkylengruppe, die durch einen gesättigten oder ungesättigten, monocyclischen oder bicyclischen Heterocyclus mit 5 bis 7 Kettengliedern, der ein oder zwei Heteroatome ausgewählt aus Sauerstoff, Stickstoff und Schwefel enthält, substituiert ist,
  * oder durch eine Gruppe der Formel $-R_{11}-CO-R_{12}$, worin $R_{11}$ die oben angegebenen Bedeutungen besitzt und $R_{12}$ eine Hydroxygruppe oder eine geradkettige oder verzweigte ($C_1$-$C_6$)-Alkoxygruppe darstellt, bedeutet,

$R_3$, $R_4$, die gleichartig oder verschieden sind, unabhängig voneinander Wasserstoffatome oder geradkettige oder verzweigte ($C_1$-$C_6$)-Alkylgruppen darstellen,

$R_5$ und/oder $R_6$ eine Gruppe der Formel -O-CO-U-V darstellen (darstellt), worin:

- U eine geradkettige oder verzweigte ($C_1$-$C_8$)-Alkylenkette, die gegebenenfalls durch eine oder mehrere, gleichartige oder verschiedene Gruppen ausgewählt aus Arylgruppen, Hydroxygruppen und geradkettigen oder verzweigten ($C_1$-$C_6$)-Alkoxygruppen substituiert ist, und
- V eine Gruppe ausgewählt aus:

  * Carboxy,
  * $-CO_2R_{13}$, worin $R_{13}$ eine geradkettige oder verzweigte ($C_1$-$C_6$)-Alkylgruppe darstellt, die gegebenenfalls durch eine oder mehrere Hydroxygruppen, Arylgruppen oder geradkettige oder verzweigte Aryl-($C_1$-$C_6$)-alkylgruppen substituiert ist,
  * Hydroxy,
  * geradkettigem oder verzweigtem ($C_1$-$C_6$)-Alkoxy,

* $-NR_7R_8$, worin $R_7$ und $R_8$, die gleichartig oder verschieden sind, die oben angegebenen Bedeutungen besitzen,
* $-NR_7-CO_2R_{13}$, worin $R_7$ und $R_{13}$ die oben angegebenen Bedeutungen besitzen,
* $-NR_7-COR_{13}$, worin $R_7$ und $R_{13}$ die oben angegebenen Bedeutungen besitzen,
* $-COR_{13}$, worin $R_{13}$ die oben angegebenen Bedeutungen besitzt und
* $-CO-NR_7R_8$, worin $R_7$ und $R_8$, die gleichartig oder verschieden sind, die oben angegebenen Bedeutungen besitzen, darstellen, bedeuten,

und in dem Fall, wo eine der beiden Gruppen $R_5$ und $R_6$ eine Gruppe der Formel -O-CO-U-V darstellt, die andere der Gruppen $R_5$ oder $R_6$ eine Gruppe Z darstellt ausgewählt aus:

- Hydroxy,
- geradkettigem oder verzweigtem ($C_1$-$C_6$)-Alkoxy,
- geradkettigem oder verzweigtem ($C_1$-$C_6$)-Alkylcarbonyloxy,
- Arylcarbonyloxy,
- geradkettigem oder verzweigtem Aryl-($C_1$-$C_6$)-alkylcarbonyloxy und
- Amino, gegebenenfalls substituiert durch eine oder zwei gleichartige oder verschiedene, geradkettige oder verzweigte ($C_1$-$C_6$)-Alkylgruppen,

deren optische Isomere, N-Oxide sowie deren Additionssalze mit einer pharmazeutisch annehmbaren Säure oder Base,
wobei es sich versteht, daß "Aryl" für eine Phenyl- oder Naphthylgruppe steht, die gegebenenfalls einen oder mehrere gleichartige oder verschiedenartige Substituenten ausgewählt aus Hydroxy, Halogen, Carboxy, Nitro, Amino, geradkettigem oder verzweigtem ($C_1$-$C_6$)-Alkylamino oder geradkettigem oder verzweigtem ($C_1$-$C_6$)-Dialkylamino, geradkettigem oder verzweigtem ($C_1$-$C_6$)-Alkoxy, geradkettigem oder verzweigtem ($C_1$-$C_6$)-Acyl und geradkettigem oder verzweigtem ($C_1$-$C_6$)-Alkylcarbonyloxy substituiert sein kann.

**2.** Verbindungen der Formel (I) nach Anspruch 1, **dadurch gekennzeichnet, daß** $R_3$ und $R_4$, die gleichartig oder verschieden sind, geradkettige oder verzweigte ($C_1$-$C_6$)-Alkylgruppen bedeuten, deren optische Isomere, N-Oxide und deren Additionssalze mit einer pharmazeutisch annehmbaren Säure oder Base.

**3.** Verbindungen der Formel (I) nach einem der Ansprüche 1 oder 2, **dadurch gekennzeichnet, daß** $R_3$ und $R_4$, die gleichartig oder verschieden sind, jeweils eine Methylgruppe bedeuten, deren optische Isomere, N-Oxide und deren Additionssalze mit einer pharmazeutisch annehmbaren Säure oder Base.

**4.** Verbindungen der Formel (I) nach Anspruch 1, **dadurch gekennzeichnet, daß** $R_2$ eine geradkettige oder verzweigte ($C_1$-$C_6$)-Alkoxygruppe oder eine gegebenenfalls substituierte Aminogruppe, wie sie bezüglich der Formel (I) definiert worden ist, bedeutet, deren optische Isomere, N-Oxide sowie deren Additionssalze mit einer pharmazeutisch annehmbaren Säure oder Base.

**5.** Verbindungen der Formel (I) nach Anspruch 1, **dadurch gekennzeichnet, daß** $R_5$ eine Gruppe der Formel -O-CO-U-V bedeutet, in der U und V die bezüglich der Formel (I) angegebenen Bedeutungen besitzen und $R_6$ eine Gruppe Z darstellt, wie sie bezüglich der Formel (I) definiert worden ist, deren optische Isomere, N-Oxide sowie deren Additionssalze mit einer pharmazeutisch annehmbaren Säure oder Base.

**6.** Verbindungen der Formel (I) nach Anspruch 1, **dadurch gekennzeichnet, daß** $R_6$ und $R_5$, die gleichartig oder verschieden sind, jeweils eine Gruppe der Formel -O-CO-U-V bedeuten, worin U und V die bezüglich der Formel (I) angegebenen Bedeutungen besitzen, deren optische Isomere, N-Oxide sowie deren Additionssalze mit einer pharmazeutisch annehmbaren Säure oder Base.

**7.** Verbindungen der Formel (I) nach einem der Ansprüche 1 oder 5, **dadurch gekennzeichnet, daß** $R_5$ eine Gruppe der Formel -O-CO-U-V darstellt, worin U eine geradkettige ($C_1$-$C_4$)-Alkylenkette und V eine Gruppe ausgewählt aus Carboxy, $-NR_7R_8$, $-NR_7-CO_2R_{13}$ und $-NR_7-COR_{13}$ darstellt, worin $R_7$, $R_8$ und $R_{13}$, die gleichartig oder verschieden sind, Wasserstoffatome oder geradkettige oder verzweigte ($C_1$-$C_6$)-Alkylgruppen bedeuten, deren optische Isomere, N-Oxide sowie deren Additionssalze mit einer pharmazeutisch annehmbaren Säure oder Base.

**8.** Verbindungen der Formel (I) nach Anspruch 1, **dadurch gekennzeichnet, daß** $R_6$ eine geradkettige oder ver-

zweigte ($C_1$-$C_6$)-Alkylcarbonyloxygruppe bedeutet, deren optische Isomere, N-Oxide sowie deren Additionssalze mit einer pharmazeutisch annehmbaren Säure oder Base.

**9.** Verbindungen der Formel (I) nach Anspruch 1, nämlich:

- (±)-cis-4-{[1-(Acetyloxy)-6-methoxy-3,3,14-trimethyl-7-oxo-2,3,7,14-tetrahydro-1*H*-benzo[b]pyrano[3,2-h] acridin-2-ylI-oxy}-4-oxobutansäure,
- (±)-*cis*-4-({1-[(3-Carboxypropanoyl)-oxy]-6-methoxy-3,3,14-trimethyl-7-oxo-2,3,7,14-tetrahydro-1*H*-benzo[*b*] pyrano[3,2-*h*]acridin-2-yl}-oxy)-4-oxobutansäure,
- (±)-*cis*-5-{[1-(Acetyloxy)-6-methoxy-3,3,14-trimethyl-7-oxo-2,3,7,14-tetrahydro-1*H*-benzo[b]pyrano[3,2-h] acridin-2-yl]-oxy}-5-oxopentansäure,
- *cis*-[1-(Acetyloxy)-6-methoxy-3,3,14-trimethyl-7-oxo-2,3,7,14-tetrahydro-1*H*benzo[*b*]pyrano[3,2-*h*]acridin-2-yl]-(dimethylamino)-acetat,
- und *cis*-[1-(Acetyloxy)-6-methoxy-3,3,14-trimethyl-7-oxo-2,3,7,14-tetrahydro-1*H*-benzo[*b*]pyrano[3,2-*h*]acridin-2-yl]-4-(dimethylamino)-butanoat,

deren optische Isomere, N-Oxide sowie deren Additionssalze mit einer pharmazeutisch annehmbaren Säure oder Base.

**10.** Verfahren zur Herstellung der Verbindungen der Formel (I) nach Anspruch 1, **dadurch gekennzeichnet, daß** man:

- *entweder ein 3-Amino-2-naphthalin-carbonsäurederivat (II):*

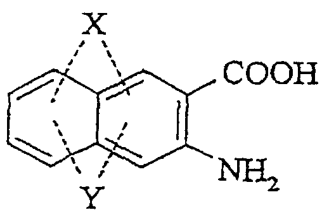

**(II)**

in der X und Y die bezüglich der Formel (I) angegebenen Bedeutungen besitzen, mit einem Phloroglucinderivat der Formel (III) umsetzt:

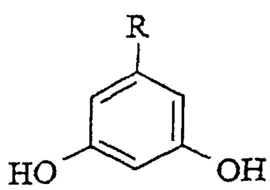

**(III)**

in der R ein Wasserstoffatom, eine Hydroxygruppe oder eine geradkettige oder verzweigte ($C_1$-$C_6$)-Alkylgruppe bedeutet,
zur Bildung der Verbindungen der Formel (IV):

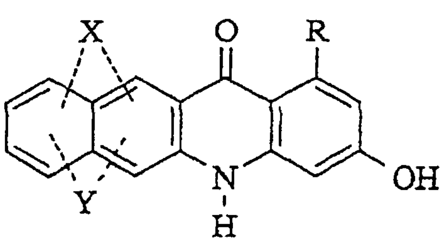

**(IV)**

in der X, Y und R die oben angegebenen Bedeutungen besitzen,
welche anschließend unter basischen Bedingungen in einem aprotischen Lösungsmittel mit einem Alkin der

Formel (V) behandelt werden:

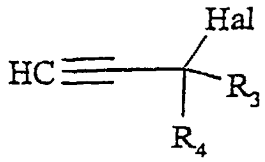

(V)

in der Hal ein Halogenatom bedeutet und $R_3$ und $R_4$ die bezüglich der Formel (I) angegebenen Bedeutungen besitzen,
zur Bildung der Verbindungen der Formel (VI):

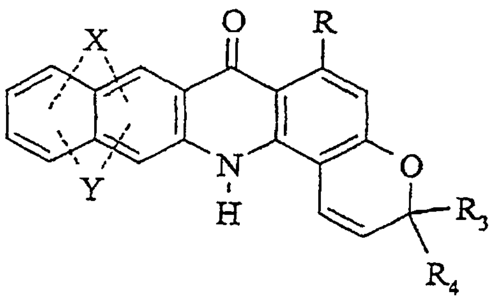

(VI)

in der X, Y, R, $R_3$ und $R_4$ die oben angegebenen Bedeutungen besitzen,

- *oder ein 3-Halogen-2-naphthalin-carbonsäurederivat der Formel (VII):*

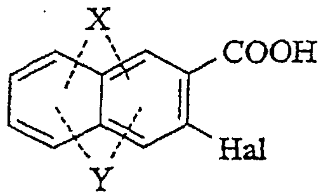

(VII)

in der X und Y die bezüglich der Formel (I) angegebenen Bedeutungen besitzen und Hal für ein Halogenatom steht,
mit einem Amino-chromenderivat der Formel (VIII) umsetzt:

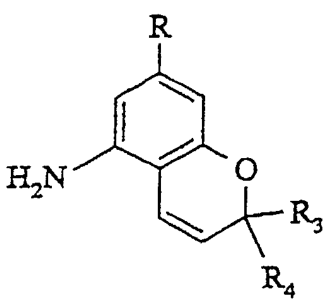

(VIII)

in der $R_3$ und $R_4$ die bezüglich der Formel (I) angegebenen Bedeutungen besitzen und R die oben angegebenen Bedeutungen aufweist,
ebenfalls zur Bildung der Verbindungen der Formel (VI):

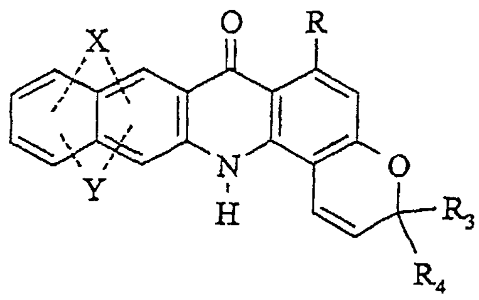

(VI)

in der X, Y, R, $R_3$ und $R_4$ die oben angegebenen Bedeutungen besitzen,
wobei bei den Verbindungen der Formel (VI) das Stickstoffatom gegebenenfalls durch Einwirkung eines Alkylhalogenids oder eines Dialkylsulfats in Gegenwart eines Deprotonierungsmittels in einem aprotischen polaren Lösungsmittel substituiert wird zur Bildung der Verbindungen der Formel (IX):

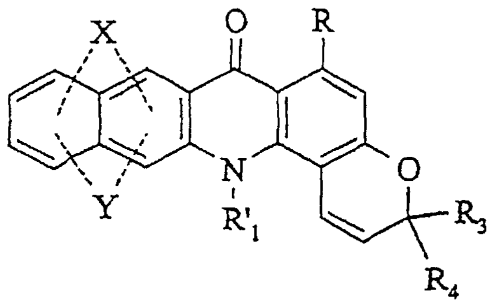

(IX)

in der X, Y, R, $R_3$ und $R_4$ die oben angegebenen Bedeutungen besitzen und $R'_1$ eine geradkettige oder verzweigte ($C_1$-$C_6$)-Alkylgruppe darstellt,
welche Verbindungen der Formel (IX) der Einwirkung eines Alkylierungsmittels, wie eines Dialkylsulfats, oder eines Acylierungsmittels unterworfen werden zur Bildung der Verbindungen der Formel (X):

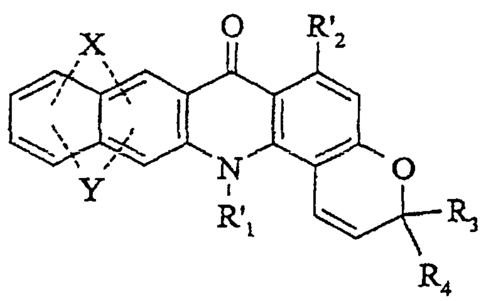

(X)

in der X, Y, $R'_1$, $R_3$ und $R_4$ die oben angegebenen Bedeutungen besitzen und $R'_2$ eine Alkoxygruppe (die gegebenenfalls durch eine Gruppe der Formel $NR_9R_{10}$ substituiert ist, in der $R_9$ und $R_{10}$ die bezüglich der Formel (I) angegebenen Bedeutungen besitzen), oder eine geradkettige oder verzweigte ($C_1$-$C_6$)-Alkylcarbonyloxygruppe bedeutet,
welche Verbindung der Formel (X) dann, wenn $R'_2$ beispielsweise eine Alkoxygruppe darstellt, mit einer Verbindung der Formel (XI) behandelt wird:

HNRaRb (XI)

in der Ra ein Wasserstoffatom, eine geradkettige oder verzweigte ($C_1$-$C_6$)-Alkylgruppe, eine Arylgruppe oder eine geradkettige oder verzweigte Aryl-($C_1$-$C_6$)-alkylgruppe und Rb ein Wasserstoffatom, eine geradkettige oder verzweigte ($C_1$-$C_6$)-Alkylgruppe, eine Arylgruppe, eine geradkettige oder verzweigte Aryl-($C_1$-$C_6$)-al-

kylgruppe, eine Gruppe der Formel $-R_{11}-NR_9R_{10}$, worin $R_{11}$, $R_{10}$ und $R_9$ die bezüglich der Formel (I) angegebenen Bedeutungen besitzen, eine geradkettige oder verzweigte $(C_1-C_6)$-Alkylcarbonylgruppe, in der der Alkylrest gegebenenfalls durch eine Gruppe $NR_7R_8$, wie sie bezüglich der Formel (I) definiert worden ist, substituiert sein kann, eine Heterocycloalkylengruppe (wobei die Begriffe Alkylen und Heterocycls die bezüglich der Formel (I) angegebenen Bedeutungen besitzen) oder eine Gruppe der Formel $-R_{11}-CO-R_{12}$, worin $R_{11}$ und $R_{12}$ die bezüglich der Formel (I) angegebenen Bedeutungen besitzen, darstellen,
zur Bildung der Verbindungen der Formel (XII):

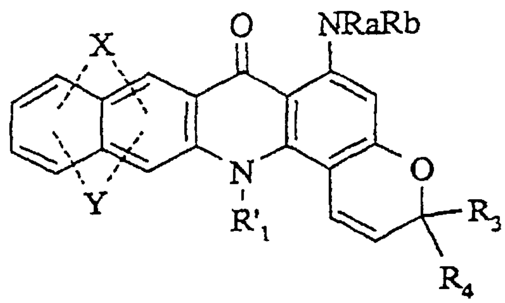

(XII)

In der X, Y, $R'_1$, $R_3$, $R_4$, Ra und Rb die oben angegebenen Bedeutungen besitzen,
wobei die Gesamtheit der Verbindungen der Formeln (VI), (IX), (X) und (XII) die Verbindungen der Formel (XIII) bilden:

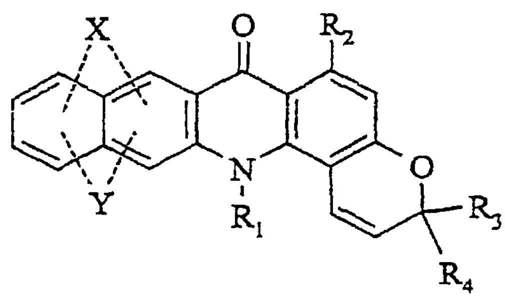

(XIII)

in der X, Y, $R_1$, $R_2$, $R_3$ und $R_4$ die bezüglich der Formel (I) angegebenen Bedeutungen besitzen, welche Verbindungen der Formel (XIII):

a) ➪ entweder der Einwirkung von Osmiumtetroxid in polarem Medium und in Gegenwart von 4-Methylmorpholin-N-oxld unterworfen werden zur Bildung der Verbindungen der Formeln (XIV/a) und (XIV/b):

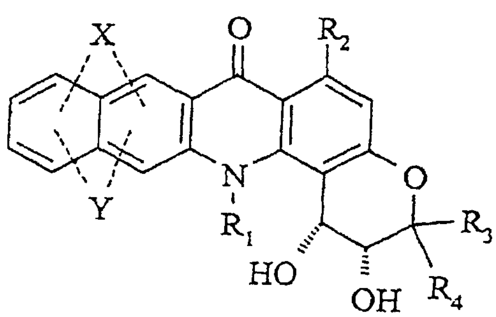

(XIV/a)

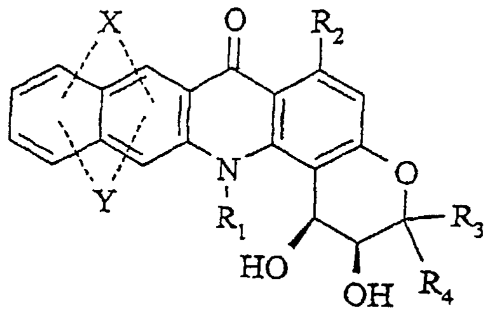

(XIV/b)

in denen X, Y, $R_1$, $R_2$, $R_3$ und $R_4$ die oben angegebenen Bedeutungen besitzen,
wobei die Verbindungen der Formeln (XIV/a) und (XIV/b) auch getrennt durch chirale Synthese erhalten werden können, insbesondere durch asymmetrische *cis*-Dihydroxylierung ausgehend von der Verbindung (XIII) unter Verwendung von chiralen Liganden,
b) ⇨ oder der Einwirkung von Kaliumpermanganat in polarem Medium unterworfen werden zur Bildung der Verbindungen der Formel (XV):

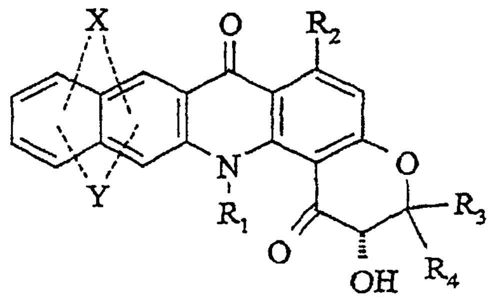

(XV)

in der X, Y, $R_1$, $R_2$, $R_3$ und $R_4$ die oben angegebenen Bedeutungen besitzen, welche Verbindungen der Formel (XV) reduzierenden Bedingungen in Gegenwart von beispielsweise $NaBH_4$ unterworfen werden zur Bildung der Verbindungen der Formel (XIV/c):

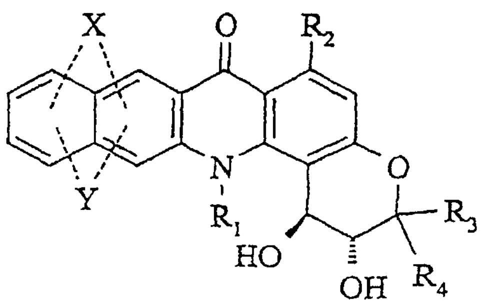

(XIV/c)

in der X, Y, $R_1$, $R_2$, $R_3$ und $R_4$ die oben angegebenen Bedeutungen besitzen,
wobei die Gesamtheit der Verbindungen der Formeln (XIV/a), (XIV/b) und (XIV/c) die Verbindungen der Formel (XIV) bildet:

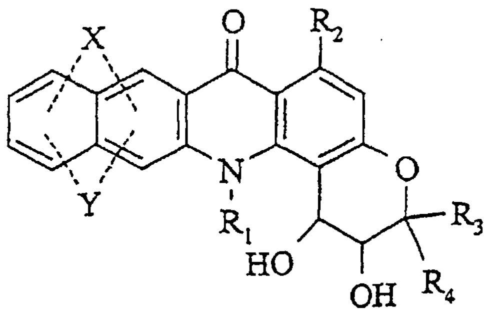

(XIV)

in der X, Y, $R_1$, $R_2$, $R_3$ und $R_4$ die oben angegebenen Bedeutungen besitzen, welche Verbindungen der Formel (XIV):

✧ entweder der Einwirkung eines Alkohols der Formel $R_{20}$-OH, in der $R_{20}$ eine geradkettige oder verzweigte ($C_1$-$C_6$)-Alkylgruppe bedeutet, unterworfen werden,
zur Bildung der Verbindungen der Formel (XVI/a):

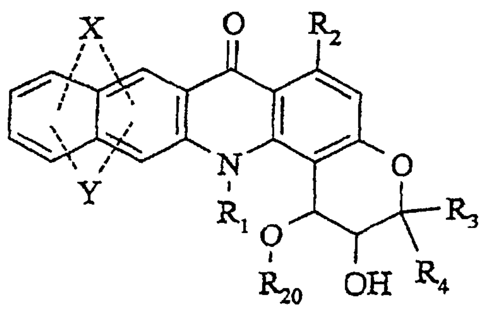

(XVI/a)

in der X, Y, $R_1$, $R_2$, $R_3$, $R_4$ und $R_{20}$ die oben angegebenen Bedeutungen besitzen,
worauf bei den Verbindungen der Formel (XVI/a) die Alkoholfunktion in Gegenwart einer Verbindung der Formel

$$V-U-\underset{\underset{O}{\|}}{C}-O-W$$

oder $(V\text{-}U\text{-}CO)_2O$, in denen U und V die bezüglich der Formel (I) angegebenen Bedeutungen besitzen und W eine austretende Gruppe darstellt, verestert wird,
zur Bildung der Verbindungen der Formel (I/a), einem Sonderfall der Verbindungen der Formel (I):

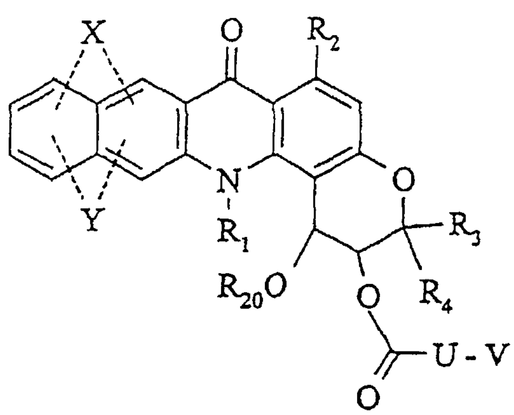

(I/a)

in der X, Y, $R_1$, $R_2$, $R_3$, $R_4$, $R_{20}$, U und V die oben angegebenen Bedeutungen besitzen,

✣ oder der Einwirkung eines Alkyliodids der Formel $R'_{20}$-I, in der $R'_{20}$ eine geradkettige oder verzweigte ($C_1$-$C_6$)-Alkylgruppe darstellt, in Gegenwart eines Silbersalzes unterworfen werden zur Bildung der Verbindungen der Formel (XVI/b):

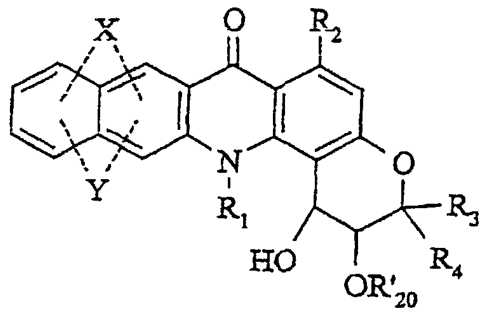

(XVI/b)

einem Sonderfall der Verbindungen der Formel (I), in der X, Y, $R_1$, $R_2$, $R_3$, $R_4$ und $R'_{20}$ die oben angegebenen Bedeutungen besitzen,

bei welchen Verbindungen der Formel (XVI/b) die Alkoholfunktion in Gegenwart einer Verbindung der Formel

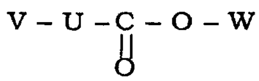

oder $(V\text{-}U\text{-}CO)_2O$, wie sie oben definiert worden sind, verestert wird, zur Bildung der Verbindungen der Formel (I/b):

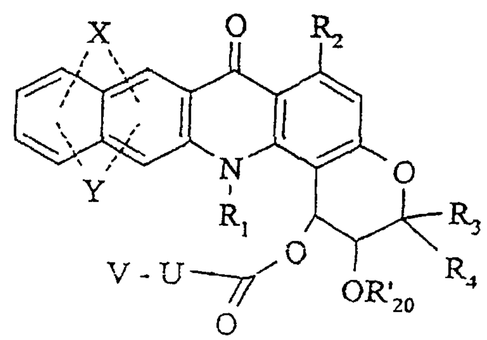

(I/b)

einem Sonderfall der Verbindungen der Formel (I), in der X, Y, $R_1$, $R_2$, $R_3$, $R_4$, $R'_{20}$, U und V die oben angegebenen Bedeutungen besitzen.

✣ oder der direkten Einwirkung einer Verbindung der Formel

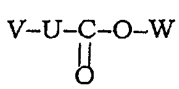

oder $(V\text{-}U\text{-}CO)_2O$, wie sie oben definiert worden sind, in Gegenwart einer Base unterworfen werden zur Bildung der Verbindungen der Formel (I/c), einem Sonderfall der Verbindungen der Formel (I):

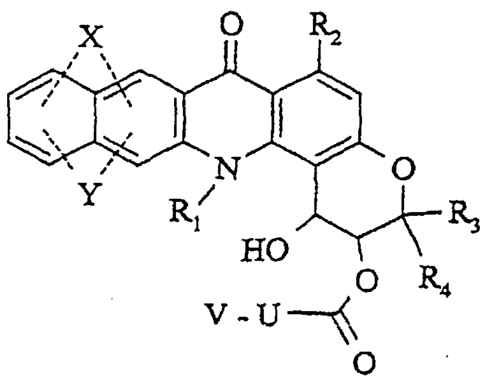

(I/c)

in der X, Y, $R_1$, $R_2$, $R_3$, $R_4$, U und V die oben angegebenen Bedeutungen besitzen, welche Verbindung der Formel (I/c) erneut unter den gleichen Verfahrensbedingungen der Einwirkung eines Anhydrids der Formel $(R_{30}CO)_2O$, in der $R_{30}$ eine geradkettige oder verzweigte ($C_1$-$C_6$)-Alkylgruppe, eine Arylgruppe oder eine geradkettige oder verzweigte Aryl-($C_1$-$C_6$)-alkylgruppe darstellt, unterworfen werden kann zur Bildung der Verbindungen der Formel (I/d):

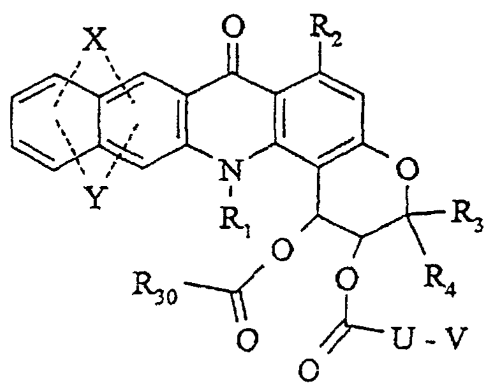

(I/d)

in der X, Y, $R_1$, $R_2$, $R_3$, $R_4$, $R_{30}$, U und V die oben angegebenen Bedeutungen besitzen,

oder welche Verbindung der Formel (I/c) erneut mit einer Verbindung der Formel

$$V-U-\underset{\displaystyle \| \atop O}{C}-O-W$$

oder $(V\text{-}U\text{-}CO)_2O$, wie sie oben definiert worden sind, behandelt werden kann zur Bildung der Verbindungen der Formel (I/e), einem Sonderfall der Verbindungen der Formel (I):

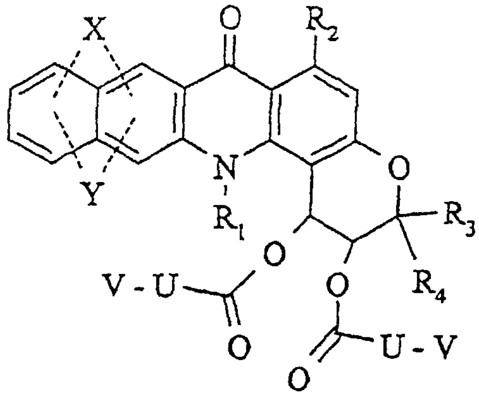

(I/e)

in der X, Y, $R_1$, $R_2$, $R_3$, $R_4$, U und V die bezüglich der Formel (I) angegebenen Bedeutungen besitzen, wobei es sich versteht, daß die beiden Grupen U und die beiden Gruppen V jeweils gleichartig oder verschieden sein können,

c) ⇨ oder der Einwirkung einer Persäure unterworfen werden können zur Bildung der Verbindungen der Formel (XVII):

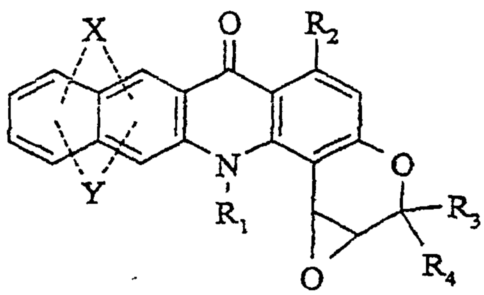

(XVII)

in der X, Y, $R_1$, $R_2$, $R_3$ und $R_4$ die oben angegebenen Bedeutungen besitzen,

welche Verbindungen der Formel (XVII) gegebenenfalls mit Ammoniak oder einem primären oder sekundären Amin behandelt werden zur Bildung in Abhängigkeit von der Art der eingesetzten Reaktionsteilnehmer der Verbindungen der Formeln (XVIII/a) und/oder (XVIII/b):

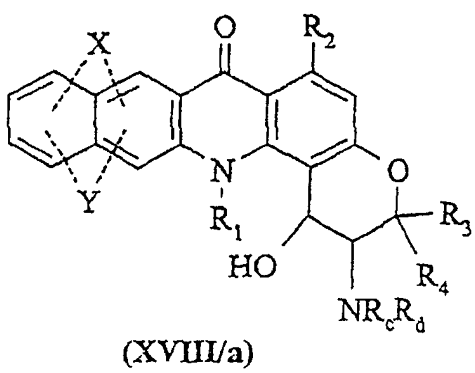

(XVIII/a)

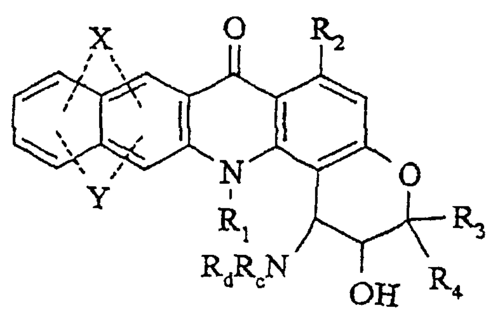

(XVIII/b)

in denen X, Y, $R_1$, $R_2$, $R_3$ und $R_4$ die oben angegebenen Bedeutungen besitzen und $R_c$ und $R_d$ ein Wasserstoffatom oder eine geradkettige oder verzweigte ($C_1$-$C_6$)-Alkylgruppe bedeuten, welche Verbindungen der Formeln (XVIII/a) und (XVIII/b) mit einer Verbindung der Formel

$$V-U-\underset{\underset{O}{\|}}{C}-O-W,$$

wie sie oben definiert worden ist, behandelt werden zur Bildung der Verbindungen der Formeln (I/f) bzw. (I/g), einem Sonderfall der Verbindungen der Formel (I):

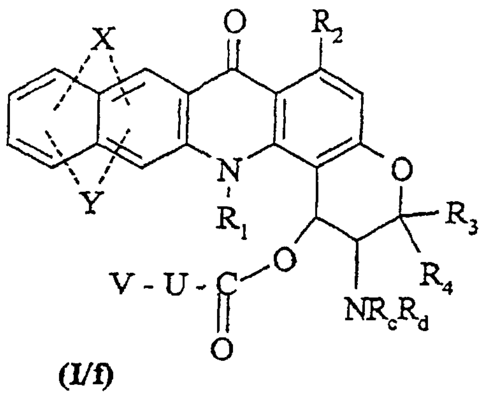

(I/f)

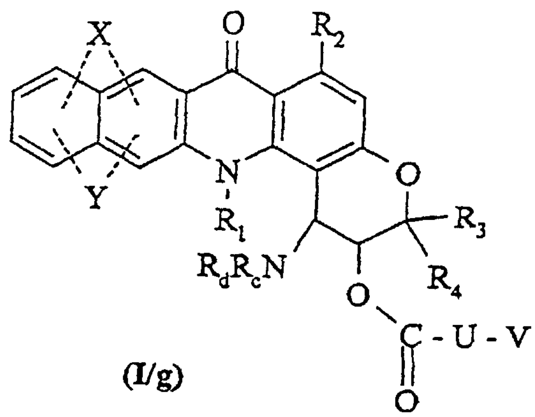

(I/g)

worin X, Y, $R_1$, $R_2$, $R_3$, $R_4$, $R_c$, $R_d$, U und V die oben angegebenen Bedeutungen besitzen, welche Verbindungen der Formeln (I/a) bis (I/g) die Gesamtheit der erfindungsgemäßen Verbindungen bilden, welche gegebenenfalls mit Hilfe einer klassischen Reinigungsmethode gereinigt werden, welche gewünschtenfalls mit Hilfe einer klassischen Trennungsmethode in ihre verschiedenen optischen Isomeren getrennt werden können und welche gewünschtenfalls in ihre N-Oxide und gewünschtenfalls in ihre Additionssalze mit einer pharmazeutisch annehmbaren Säure oder Base umgewandelt werden.

**11.** Pharmazeutische Zubereitungen enthaltend als Wirkstoff mindestens eine Verbindung nach einem der Ansprüche 1 bis 9 allein oder in Kombination mit einem oder mehreren inerten, nichttoxischen, pharmazeutisch annehmbaren Trägermaterialien oder Bindemitteln.

**12.** Pharmazeutische Zubereitungen nach Anspruch 11 enthaltend mindestens einen Wirkstoff nach einem der Ansprüche 1 bis 9 zur Behandlung von Krebs.